(19)

**Europäisches Patentamt**
**European Patent Office**
**Office européen des brevets**

(11) **EP 4 616 864 A1**

(12) **EUROPEAN PATENT APPLICATION**

(43) Date of publication:
**17.09.2025 Bulletin 2025/38**

(21) Application number: **24382257.4**

(22) Date of filing: **11.03.2024**

(51) International Patent Classification (IPC):
***A61K 38/16*** *(2006.01)*     ***A61K 45/06*** *(2006.01)*
***A61P 25/28*** *(2006.01)*     ***A61P 37/06*** *(2006.01)*

(52) Cooperative Patent Classification (CPC):
**A61K 38/16; A61K 45/06; A61P 25/28; A61P 37/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Peptomyc, S.L.**
  **08035 Barcelona (ES)**
- **Fundació Privada Institut d'Investigació Oncològica de Vall Hebron**
  **08035 Barcelona (ES)**
- **Institució Catalana de Recerca i Estudis Avançats**
  **08010 Barcelona (ES)**
- **Fundació Hospital Universitari Vall d'Hebron - Institut de Recerca**
  **08035 Barcelona (ES)**

(72) Inventors:
- **CASACUBERTA SERRA, Sílvia**
  **E-08500 Vic (ES)**
- **BEAULIEU, Marie-Eve**
  **E-08023 Barcelona (ES)**
- **SOUCEK, Laura**
  **E-08006 Barcelona (ES)**
- **ESPEJO RUIZ, Carmen**
  **E-08960 Sant Just Desvern (ES)**
- **EIXARCH AHUFINGER, Herena**
  **E-08042 Barcelona (ES)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

(54) **COMPOUND FOR THE TREATMENT OF AUTOIMMUNE DISEASES**

(57)     The present invention relates to a polypeptide comprising Omomyc, a functionally equivalent variant thereof, a conjugate comprising Omomyc or said functionally equivalent variant, a polynucleotide encoding said polypeptides, a vector comprising said polynucleotide, and a cell capable of secreting the polypeptide or the conjugate for use in the prevention and/or treatment of an autoimmune disease. The invention also relates to combinations and pharmaceutical compositions of said compounds and at least a further compound useful in the treatment of an autoimmune disease, as well as their medical uses.

EP 4 616 864 A1

**Description**

FIELD OF THE INVENTION

The present invention relates to the field of autoimmune diseases and, more particularly, to a compound and its use in the prevention and/or treatment of autoimmune diseases.

BACKGROUND OF THE INVENTION

The aberrant regulation of immune reactions has been associated with a wide array of human diseases, since the inappropriate mounting of an immune response against various self and foreign antigens plays a causal role in a huge number of pathologies including autoimmune disorders, asthma, allergic reactions, graft-versus-host disease, transplantation graft rejection and a variety of other immunological disorders.

Autoimmune diseases form a group of diseases caused by an impairment of the immune system that makes it react against the body's own tissues.

The modification of the cell recognition mechanisms that normally enable the body to distinguish between the "self" and the "non-self", i.e. between elements that belong to the body and elements that are foreign to it, give rise to the production of antibodies and differentiation of specific T and B cells which can target single organs (organ-specific diseases) or trigger systemic disorders, damaging the individual's functions as a whole.

In particular, multiple sclerosis (MS) is a chronic immune-mediated disease that affects the central nervous system (CNS) and is characterized by the destruction of the myelin sheet, axonal damage and neuronal loss leading to neurological disability. The prevalence of the disease is increasing, and actually up to 2.8 million people worldwide have been diagnosed with MS. In fact, it is considered the first non-traumatic cause of disability in young adults.

The majority of studies on the pathogenesis of MS have been performed in the experimental autoimmune encephalomyelitis (EAE) animal model, which can be induced by injecting susceptible rodent strains with myelin proteins or peptides. These animals subsequently develop either a monophasic inflammatory CNS disease or various forms of chronic or relapsing-remitting EAE and, in their CNS lesions, CD4+ T cells, monocytes/activated microglia and other cell populations and humoral factors are found (Martin R et al. Immunological aspects of demyelinating diseases. Ann Rev Immunol. 1992, 10:153-187).

The etiology of MS is still unknown, however, several genetic, environmental and life-style factors have been linked to disease risk. The most accepted hypothesis for disease pathogenesis relies on the activation in the periphery of CNS-antigen autoreactive T cells in front of CNS-derived antigens or antigens from pathogens that present high homology with CNS antigens. CNS-autoreactive T cells then circulate into the CNS and are reactivated by resident antigen presenting cells (APCs), which present CNS-antigens to autoreactive T cells, giving rise to a series of immune events that ultimately cause neurodegeneration and neurological disability. Since MS is an immune-mediated disease, current available treatments are devoted to downregulate the peripheral immune response by both inhibiting and modulating it (immunosuppressors or immunomodulators).

In general, treatment of autoimmune diseases depends on the type and severity of the condition. Nonsteroidal anti-inflammatory drugs (NSAIDs), steroids and immunosuppressants are often used. Intravenous immunoglobulin may also occasionally be used. For instance, in the case of multiple sclerosis, interferon (IFN)-beta, glatiramer acetate, cyclophosphamide or mitoxantrone are typically employed. Attempts at providing new treatments against autoimmune diseases include the use of proteins or small organic compounds as immunomodulators.

However, while reported treatments usually improve symptoms they do not typically cure the disease, and in some cases side-effects of the treatment can be just as deleterious as the disease itself. In particular, genotoxic side-effects, such as clastogenicity or aneugenicity, which are typically associated to treatments that interfere with epigenetic regulation, are of particular importance due to their seriousness and potential lethality.

In summary, there is a constant and urgent need to develop new treatments against autoimmune diseases which enrich the plethora of existing therapies and preferably overcome drawbacks associated to existing therapies, such as the use of a high dose and high frequency of administration.

SUMMARY OF THE INVENTION

The authors of the present invention have demonstrated that the inhibition of Myc using the polypeptide Omomyc constitutes a potential therapeutic option for treating autoimmune diseases. They have found that Omomyc reduces splenocyte proliferation and induces cell death in a dose dependent manner in splenocytes from EAE-diseased mice (Figure 1). It has also been found that the treatment of EAE-diseased mice with Omomyc delays disease onset and ameliorates the clinical course of the disease when administered by different routes and at different dose and intervals (Figures 2-4). Surprisingly, the beneficial effect of Omomyc is long lasting since only four doses are enough to achieve the therapeutic effect (Figure 4).

Thus, in a first aspect, the present invention relates to a compound, hereinafter the compound of the invention, selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or the functionally equivalent variant thereof,
(c) a polynucleotide encoding the polypeptide of a) or the conjugate of b),
(d) a vector comprising the polynucleotide according to c), and
(e) a cell capable of secreting into the medium the polypeptide according to a) or the conjugate according to b)

for use in the prevention and/or treatment of an autoimmune disease in a subject.

In a second aspect, the invention relates to a combination comprising:

(i) a compound selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,
(c) a polynucleotide encoding the polypeptide of a) or the conjugate of b),
(d) a vector comprising the polynucleotide according to c), and
(e) a cell capable of secreting into the medium the polypeptide according to a) or the conjugate according to b); and

(ii) an agent useful in the treatment of an autoimmune disease, preferably selected from the group consisting of copaxone, teriflunomide, fumarate, natalizumab, fingolimod, ocrelizumab, ponesimod, siponimod, ozanimod, and tocilizumab.

In a third aspect, the invention relates to a pharmaceutical composition comprising a pharmaceutically effective amount of a combination of the second aspect of the invention and a pharmaceutically acceptable excipient.

In a fourth aspect, the invention relates to a combination of the second aspect of the invention or a pharmaceutical composition of the third aspect of the invention for use in medicine.

In a fifth aspect, the invention relates to a combination of the second aspect of the invention or a pharmaceutical composition according to the third aspect of the invention for use in the prevention and/or treatment of an autoimmune disease.

## BRIEF DESCRIPTION OF THE DRAWINGS

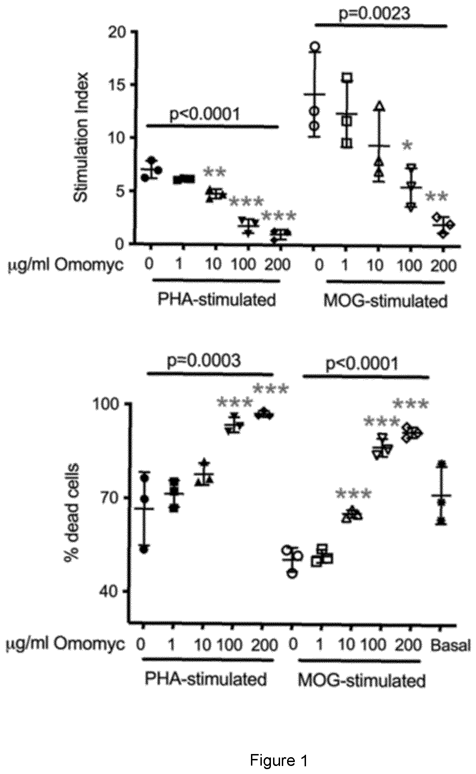

**Figure 1.** *Omomyc reduces splenocyte proliferation and induces cell death in a dose dependent manner, both in* phytohematoagglutinin *(PHA)- and* myelin oligodendrocyte glycoprotein *(MOG)-stimulated splenocytes from* Experimental autoimmune encephalomyelitis *(EAE)-diseased mice.* EAE was induced to C57BL6/J mice. When mice had already developed neurological disability, splenocytes were isolated and cultured in the presence of PHA or the 35-55 MOG peptide with increasing concentrations of Omomyc. Proliferation was measured by incorporation of $^3$H-thymidine and cell death by flow cytometry.

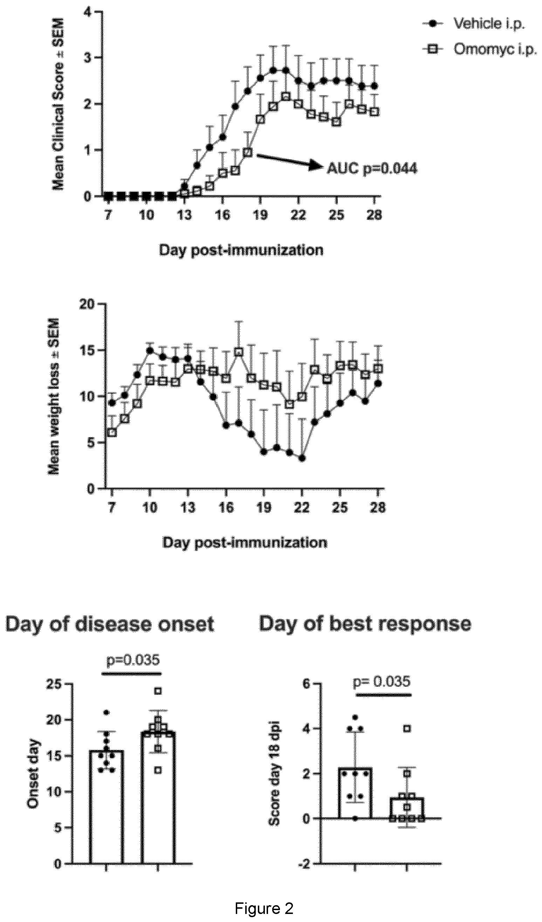

**Figure 2.** *Omomyc treatment delays disease onset and ameliorates the clinical course of the disease.* Experimental autoimmune encephalomyelitis (EAE) was induced to C57BL6/J mice and clinical evolution was followed daily. Omomyc was administered daily at 50mg/kg via intraperitoneal (i.p.) injection from day 1 post-immunization. SEM, standard error of mean; AUC, area under the curve; dpi, day post-immunization.

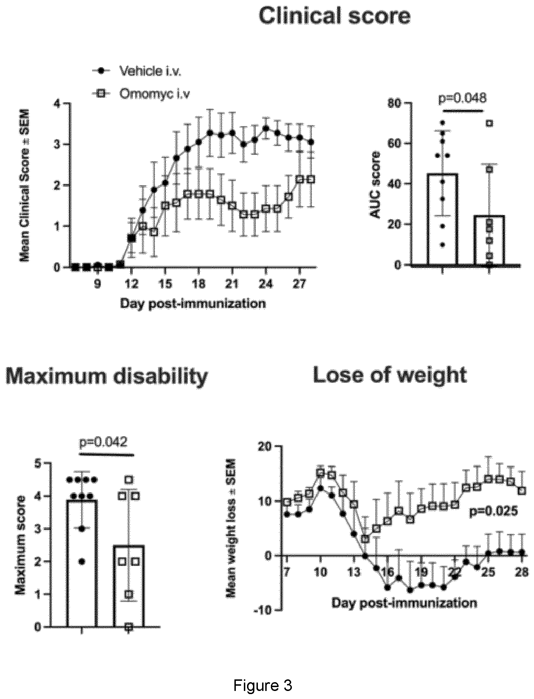

**Figure 3.** *Omomyc treatment significantly ameliorates the clinical course of the disease with animals displaying milder clinical signs (decrease of maximum score).* Experimental autoimmune encephalomyelitis (EAE) was induced to C57BL6/J mice and clinical evolution was followed daily. Omomyc was administered thrice per week at 75mg/kg intravenously (i.v.) from day 1 post-immunization.

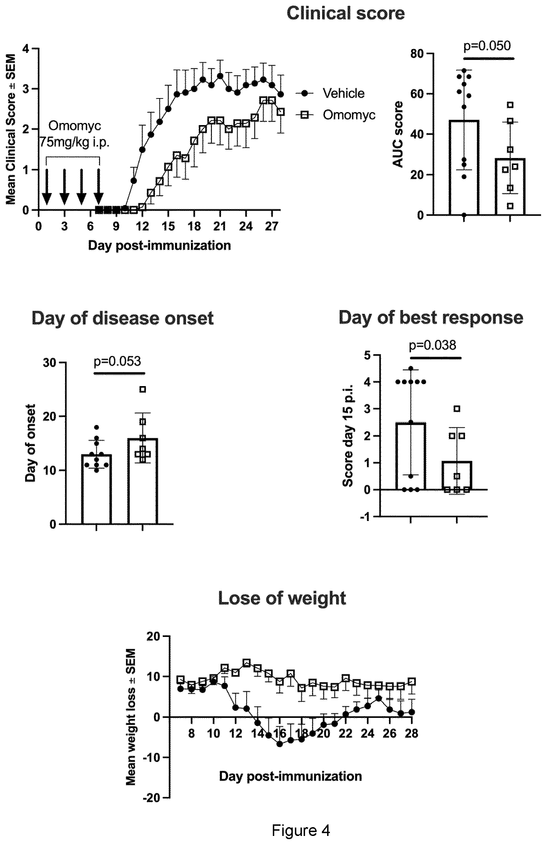

**Figure 4.** *The beneficial effect of Omomyc is long lasting.* Experimental autoimmune encephalomyelitis (EAE) was induced to C57BL6/J mice and clinical evolution was followed daily. Four doses of 75mg/kg Omomyc administered intraperitoneally were given every other day starting at day 1 post-immunization. Only four doses of Omomyc were able to delay disease onset and to ameliorate the clinical course of the disease. Omomyc-treated animals did not show any weight loss compared to their vehicle-treated counterparts, which displayed a significant weight loss after the onset of the clinical signs.

## DETAILED DESCRIPTION OF THE INVENTION

The present invention relates to a therapeutic compound for use in the prevention and/or treatment of autoimmune diseases in a subject.

Unless otherwise defined, all technical terms used herein have the same meaning as commonly understood by one of ordinary skill in the art to which this invention belongs.

Therapeutic uses of the invention

In a first aspect, the present invention relates to a compound selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or the functionally equivalent variant thereof,
(c) a polynucleotide encoding the polypeptide of a) or the conjugate of b)
(d) a vector comprising the polynucleotide according to c), and
(e) a cell capable of secreting into the medium the polypeptide according to a) or the conjugate according to b)

for use in the prevention and/or treatment of an autoimmune disease in a subject.

Alternatively, the present invention relates to the use of a compound selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or the functionally equivalent variant thereof,
(c) a polynucleotide encoding the polypeptide of a) or the conjugate of b)
(d) a vector comprising the polynucleotide according to c), and
(e) a cell capable of secreting into the medium the polypeptide according to a) or the conjugate according to b)

for the manufacture of a medicament for the prevention and/or treatment of an autoimmune disease in a subject.

Alternatively, the present invention relates to a method for the prevention and/or treatment of an autoimmune disease that comprises administering to a subject in need thereof a therapeutically effective amount of a compound selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or the functionally equivalent variant thereof,
(c) a polynucleotide encoding the polypeptide of a) or the conjugate of b)
(d) a vector comprising the polynucleotide according to c), and

a cell capable of secreting into the medium the polypeptide according to a) or the conjugate according to b).In a preferred embodiment, the compound for use according to the invention is a polypeptide, hereinafter the polypeptide of the invention, comprising the sequence SEQ ID NO: 1 or a functionally equivalent variant thereof.

In a more preferred embodiment, the compound for use according to the invention is a polypeptide comprising the sequence SEQ ID NO: 1.

The terms "polypeptide" and "peptide" are used interchangeably herein to refer to polymers of amino acids of any length. The polypeptide of the invention can comprise modified amino acids, and it can be interrupted by non-amino acids. In a preferred embodiment the polypeptide is exclusively formed by amino acids. Preferably the polypeptide that forms item (a) has a length between 80 and 500 amino acids, more preferably between 80 and 300 amino acids, more preferably between 80 and 250 amino acids, more preferably between 80 and 150, even more preferably between 80 and 130 amino acids, preferably between 90 and 130 amino acids, preferably no more than 125 amino acids, more preferably no more than 100 amino acids. In a preferred embodiment, the polypeptide has a length between 90 and 98 amino acids, preferably between 90 and 95 amino acids, more preferably 91 amino acids.

The term "amino acid" refers to naturally occurring and synthetic amino acids, as well as amino acid analogs and amino acid mimetics that function in a manner similar to the naturally occurring amino acids. Furthermore, the term "amino acid" includes both D- and L-amino acids (stereoisomers). Preferably, the amino acids are L-amino acids.

The term "natural amino acids" or "naturally occurring amino acids" comprises the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, nor-leucine and ornithine.

As used herein, the term "non-natural amino acid" or "synthetic amino acid" refers to a carboxylic acid, or a derivative thereof, substituted at position "a" with an amine group and being structurally related to a natural amino acid. Illustrative non- limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxy lysine, alio hydroxy lysine, 3-hydroxyproline, 4-hydroxyproline, isodesmosine, alloisoleucine, N-methylglycine, N-methylisoleucine, 6-N-methyl-lysine, N-methylvaline, norvaline, norleucine, ornithine, etc.

The polypeptide for use according to the present invention may also comprise non-amino acid moieties, such as for example, hydrophobic moieties (various linear, branched, cyclic, polycyclic or heterocyclic hydrocarbons and hydrocarbon derivatives) attached to the peptides; various protecting groups which are attached to the compound's terminals to decrease degradation. Suitable protecting functional groups are described in Green and Wuts, "Protecting Groups in Organic Synthesis", John Wiley and Sons, Chapters 5 and 7, 1991.

Chemical (non-amino acid) groups present in the polypeptide may be included in order to improve various physiological properties such as decreased degradation or clearance; decreased repulsion by various cellular pumps, improve various modes of administration, increased specificity, increased affinity, increased stability, bioavailability, solubility, decreased toxicity and the like.

"Mimetic" include molecules which mimic the chemical structure of a peptidic structure and retain the functional properties of the peptidic structure. Approaches to designing peptide analogs, derivatives and mimetics are known in the art.

In an embodiment, the polypeptide for use according to the invention is a polypeptide consisting of sequence SEQ ID NO: 1 or a polypeptide consisting of a functionally equivalent variant of SEQ ID NO: 1, preferably is a polypeptide consisting of the sequence SEQ ID NO: 1.

The SEQ ID NO: 1 corresponds to

TEENVKRRTHNVLERQRRNELKRSFFALRDQIPELENNEKAPKVVILKKATAYILSVQA

ETQKLISEIDLLRKQNEQLKHKLEQLRNSCA (SEQ ID NO: 1).

The polypeptide of sequence SEQ ID NO: 1 corresponds to the Omomyc protein sequence. The term "Omomyc", as used herein, refers to a polypeptide which consists of a mutated version of the bHLHZip domain of the Myc carrying the E61T, E68I, R74Q and R75N mutations (wherein the numbering of the mutated positions is given with respect to the sequence of Myc region corresponding to amino acids 365-454 of the polypeptide as defined under accession number NP_002458 in the NCBI database, release of March 15, 2015). The sequence of c-Myc provided in the NCBI database under the accession number NP_002458 is shown below (SEQ ID NO: 2), wherein the region from which Omomyc derives is shown underlined:

```
  1 MDFFRVVENQ QPPATMPLNV SFTNRNYDLD YDSVQPYFYC DEEENFYQQQ QQSELQPPAP

 61 SEDIWKKFEL LPTPPLSPSR RSGLCSPSYV AVTPFSLRGD NDGGGGSFST ADQLEMVTEL

121 LGGDMVNQSF ICDPDDETFI KNIIIQDCMW SGFSAAAKLV SEKLASYQAA RKDSGSPNPA

181 RGHSVCSTSS LYLQDLSAAA SECIDPSVVF PYPLNDSSSP KSCASQDSSA FSPSSDSLLS

241 STESSPQGSP EPLVLHEETP PTTSSDSEEE QEDEEEIDVV SVEKRQAPGK RSESGSPSAG

301 GHSKPPHSPL VLKRCHVSTH QHNYAAPPST RKDYPAAKRV KLDSVRVLRQ ISNNRKCTSP

361 RSSDTEENVK RRTHNVLERQ RRNELKRSFF ALRDQIPELE NNEKAPKVVI LKKATAYILS

421 VQAEEQKLIS EEDLLRKRRE QLKHKLEQLR NSCA
```
(SEQ ID NO: 2)

Omomyc also contains the M2 domain of c-Myc, having the sequence RQRRNELKRSF (SEQ ID NO: 3) (see Dang and Lee, Mol.Cell. Biol., 1988, 8:4048-4054) (double underlined above), and which corresponds to a nuclear localization signal.

Omomyc is characterized in that it shows increased dimerization capacity with all three oncogenic Myc proteins (c-Myc, N-Myc and L-Myc). Omomyc can derive from the bHLHZip domain of any Myc protein known in the art, provided that the mutations which result in the Myc suppressor effect are preserved. Thus, the Omomyc that can be used in the present invention may derive from any mammal species, including but not being limited to domestic and farm animals (cows, horses, pigs, sheep, goats, dog, cats or rodents), primates and humans. Preferably, the Omomyc protein is derived from human Myc protein (accession number NP_002458, release of March 12, 2019).

The term "Myc", as used herein, refers to a family of transcription factors which includes c-Myc, N-Myc and L-Myc. Myc protein activates expression of many genes through binding on consensus sequence CACGTG (Enhancer Box sequences or E-boxes and recruiting histone acetyl-transferases or HATs). However, Myc can also act as a transcriptional repressor. By binding the Miz-1 transcription factor and displacing p300 co-activator, it inhibits expression of Miz-1 target genes. Myc also has a direct role in the control of DNA replication.

The Myc b-HLH-LZ or Myc basic region helix-loop-helix leucine zipper domain refers to a region which determines Myc dimerization with Max protein and binding to Myc-target genes. This region corresponds to amino acids 365-454 of human Myc and is characterized by two alpha helices connected by a loop (Nair, S. K., & Burley, S. K., 2003, Cell, 112: 193-205).

In a preferred embodiment, the polypeptide of the invention is a polypeptide that comprises, consists of or consists essentially of the SEQ ID NO: 4 shown below.

MTEENVKRRTHNVLERQRRNELKRSFFALRDQIPELENNEKAPKVVILKKATAYILSVQ

AETQKLISEIDLLRKQNEQLKHKLEQLRNSCA (SEQ ID NO: 4).

In this context, "consisting essentially of" means that the specified molecule would not contain any additional sequences that would alter the activity of SEQ ID NO: 4.

Preferably, the polypeptide consists of SEQ ID NO: 4.

The term "functionally equivalent variant", refers to any polypeptide which results from the insertion or addition of one or more amino acids and/or from the deletion of one or more amino acids and/or from the conservative substitution of one or more amino acids with respect to the polypeptide of SEQ ID NO: 1 and/or which results from the chemical modification of the polypeptide of SEQ ID NO: 1 and which substantially preserves the suppressor activity of the SEQ ID NO: 1. Preferably, the functionally equivalent variant refers to any polypeptide which results from the insertion or addition of one or more amino acids and/or from the deletion of one or more amino acids and/or from the conservative substitution of one or more amino acids with respect to the polypeptide of SEQ ID NO: 1 and which substantially preserves the suppressor activity of SEQ ID NO: 1; more preferably results from the insertion or addition of one or more amino acids with respect to the polypeptide of SEQ ID NO: 1.

The skilled person will understand that the preservation of the suppressor activity requires that the variant can dimerize with Myc and/or its obligate partner p21/p22Max and inhibit Myc activity, that is capable of translocating across the cell membrane and that is capable of translocating across the nuclear envelope. In some embodiments, the functionally equivalent variant of the polypeptide of the invention homodimerizes less than Omomyc, or is not forced into homodimers by the formation of disulphide bridge. In particular the disulphide bridge formation in the homodimer form of certain embodiments of the polypeptide of the invention is less than in the polypeptide Omomyc.

"Less homodimerization", as used herein, relates to the lower ability of forming obligate homodimers of the polypeptide of the invention even in reducing conditions. In a preferred embodiment, the ability is at least 5%, at least 10%, at least 15%, at least 20%, at least 25%, at least 30%, at least 35%, at least 40%, at least 45 %, at least 50%, at least 55%, at least 60%, at least 65%, at least 70%, at least 75%, at least 80%, at least 85%, at least 90%, at least 95% less than the ability of forming homodimers of Omomyc.

Reducing conditions, as used herein, relates to the presence of a reducing agent, a compound that donates an electron to another chemical species in a redox chemical reaction. Illustrative, non-limitative examples of reducing agents are DTT (dithiothreitol), b-mercaptoethanol or TCEP (tris(2-carboxyethyl)phosphine). It is possible that the amount of homodimers is the same *in vitro,* and that the difference between the functionally equivalent variant and Omomyc is present only in cells in presence of heterodimerization partners where the absence of the disulfide enables a potentially higher formation of heterodimers.

Several assays may be used to determine the homodimerization of a peptide, by way of illustrative non-limitative example by thermal denaturation monitored by Circular dichroism, so dimerization may be detected through folding and thermal stability quantification.

Suitable functionally equivalent variants include polypeptides consisting essentially of the polypeptide of SEQ ID NO: 1. In this context, "consisting essentially of" means that the specified molecule would not contain any additional sequences that would alter the activity of the SEQ ID NO: 1.

In a preferred embodiment, the functionally equivalent variant of SEQ ID NO: 1 is a polypeptide which results from the insertion or addition of one or more amino acids with respect to the polypeptide of SEQ ID NO: 1. In an embodiment, the functionally equivalent variant results from the insertion of less than 10 amino acids, more preferably less than 5 amino acids, more preferably results from the insertion of one amino acid. In a preferred embodiment, results from the insertion of one amino acid that is methionine.

In another embodiment, the functionally equivalent variant of SEQ ID NO: 1 is a polypeptide which results from the deletion of one or more amino acids with respect to the polypeptide of SEQ ID NO: 1. In an embodiment, the functionally equivalent variant results from the deletion of less than 10 amino acids, more preferably less than 5 amino acids, more preferably results from the deletion of one amino acid.

Suitable functional variants of the targeting polypeptide are those showing a degree of identity with respect to the peptide of SEQ ID NO:1 of about greater than 25% amino acid sequence identity, such as 25%, 30%, 40%, 50%, 60%, 65%, 70%, 75%, 80%, 85%, 90%, 91%, 92%, 93%, 94%, 95%, 96%, 97%, 98% or 99%. The degree of identity between two polypeptides is determined using computer algorithms and methods that are widely known for the persons skilled in the art. The identity between two amino acid sequences is preferably determined by using the BLASTP algorithm as described previously (BLAST Manual, Altschul, S., et al., NCBI NLM NIH Bethesda, Md. 20894, Altschul, S., et al., J. Mol. Biol. 1990; 215: 403-410). In a preferred embodiment, the sequence identity is determined throughout the whole length of the polypeptide of SEQ ID NO: 1 or throughout the whole length of the variant or of both.

The functionally equivalent variants of the polypeptide of the invention may also include post-translational modifications, such as glycosylation, acetylation, isoprenylation, myristoylation, proteolytic processing, etc.

In another embodiment, suitable functional variants of the targeting peptide are those wherein one or more positions within the polypeptide for use according to the invention contain an amino acid which is a conservative substitution of the amino acid present in the protein mentioned above. "Conservative amino acid substitutions" result from replacing one amino acid with another having similar structural and/or chemical properties. For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example, by Dordo et al., (J. Mol. Biol, 1999, 217; 721-739) and Taylor et al., (J. Theor. Biol., 1986, 119:205-218).

It will be understood that in a preferred embodiment the functionally equivalent variants of Omomyc contain mutations at positions corresponding to E61, E68, R74 and R75 found in Omomyc derived from human c-Myc, preferably contain mutations at positions corresponding to the mutations E61T, E68I, R74Q and R75N found in Omomyc derived from human c-Myc. The position wherein said mutations have to occur in the functionally equivalent variant can be determined by a multiple sequence alignment of different Myc sequences and identified by the alignment of those positions corresponding to positions 61, 68, 74 and 75 within the sequence of Omomyc derived from human c-Myc. In an embodiment, the functionally equivalent variants of Omomyc contain mutations at positions corresponding to the mutations E61T, E68I, R74Q and R75N found in Omomyc derived from human c-Myc.

In another embodiment, the functionally equivalent variants of Omomyc contain mutations at positions corresponding to E61, E68, R74 and R75 within the sequence of Omomyc wherein E61 has been mutated to E61A or E61S; E68 has been mutated to E68L, E68M or E68V; R74 has been mutated to R74N; and R75 has been mutated to R75Q.

A multiple sequence alignment is an extension of pairwise alignment to incorporate more than two sequences at a time. Multiple alignment methods align all of the sequences in a given query set. A preferred multiple sequence alignment program (and its algorithm) is ClustalW, Clusal2W or ClustalW XXL (see Thompson et al. (1994) Nucleic Acids Res 22:4673-4680). Once the sequences of c-Myc from different organisms and of the variant are compared (aligned) as described herein, the skilled person can readily identify the positions within each of the sequence corresponding to positions E61T, E68I, R74Q and R75N found in Omomyc and introduce within the Omomyc variant mutations corresponding to the E61T, E68I, R74Q and R75N mutations found in Omomyc derived from human c-Myc.

Suitable assays for determining whether a polypeptide can be considered as a functionally equivalent variant of Omomyc include, without limitation:

- Assays which measure the capacity of the polypeptide to form dimeric complexes with Max and Myc, such as the assays based on the expression of a reporter gene as described in Soucek et al. (Oncogene, 1998, 17: 2463 - 2472) as well as PLA (protein Ligation assay) or Co-immunoprecipitation.

- Assays which measure the capacity of the polypeptide to bind to the Myc/Max recognition site within DNA (the CACGTG site), such as the electrophoretic mobility shift assay (EMSA) described in Soucek et al. (supra.)

- Assays which measure the capacity to repress Myc-induced transactivation, such as the assay based on the expression of a reporter gene under the control of the DNA binding sites specific for Myc/Max as described by Soucek et al. (supra.).

- Assays based on the capacity of the polypeptide to inhibit growth of cells expressing Myc, as described by Soucek et al. (supra).

- Assays which measure the ability of the polypeptide to enhance Myc-induced apoptosis, such as the assays described by Soucek et al. (Oncogene, 1998: 17, 2463 - 2472). Moreover, any assay commonly known in the art for assessing apoptosis in a cell can be used, such as the Hoechst staining, Propidium Iodide (PI) or Annexin V staining, trypan blue, DNA laddering/fragmentation and TUNEL.

- Assays which measure the ability of the polypeptide to reduce proliferation and induce cell death in splenocytes from EAE-diseased mice as described in the examples of the present invention.

- Assays which measure the ability of the polypeptide to delay disease onset and ameliorate the clinical course of the disease of EAE as described in the examples of the present invention.

In a preferred embodiment, a polypeptide is considered a functionally equivalent variant of Omomyc if it shows an activity in one or more of the above assays which is at least 10%, 20%, 30%, 40%, 50%, 60%, 70%, 80%, 90% or 100% of the native Omomyc.

In a particular embodiment, the functionally equivalent variant of the polypeptide of SEQ ID NO: 1 comprises the polypeptide of SEQ ID NO: 1, wherein the residue X at position 89 of SEQ ID NO: 1 is not a cysteine. Preferably, the residue X at position 89 of SEQ ID NO: 1 is an aliphatic amino acid, or a sulfured amino acid, or a dicarboxylic amino acid or their amides, or an amino acid having two basic groups, or an aromatic amino acid, or a cyclic amino acid, or a hydroxylated amino acid. More preferably is an amino acid selected from serine, threonine and alanine, preferably selected from serine and alanine.

Suitable functionally equivalent variants of SEQ ID NO: 1 having a residue X at position 89 of SEQ ID NO: 1 which is not a cysteine are disclosed in the following table.

| SEQ ID NO | SEQUENCE |
|---|---|
| SEQ ID NO: 5 | TEENVKRRTHNVLERQRRNELKRSFFALRDQIPELENNEKAPKVVILKKATA YILSVQAETQKLISEIDLLRKQNEQLKHKLEQLRNSXA (wherein X is any aa different from Cys) |
| SEQ ID NO: 6 | MTEENVKRRTHNVLERQRRNELKRSFFALRDQIPELENNEKAPKVVILKKAT AYILSVQAETQKLISEIDLLRKQNEQLKHKLEQLRNSXA (wherein X is any aa different from Cys) |

(continued)

| SEQ ID NO | SEQUENCE |
|---|---|
| SEQ ID NO: 7 | TEENVKRRTHNVLERQRRNELKRSFFALRDQIPELENNEKAPKVVILKKATA YILSVQAETQKLISEIDLLRKQNEQLKHKLEQLRNSSA |
| SEQ ID NO: 8 | MTEENVKRRTHNVLERQRRNELKRSFFALRDQIPELENNEKAPKVVILKKAT AYILSVQAETQKLISEIDLLRKQNEQLKHKLEQLRNSSA |
| SEQ ID NO: 9 | TEENVKRRTHNVLERQRRNELKRSFFALRDQIPELENNEKAPKVVILKKATA YILSVQAETQKLISEIDLLRKQNEQLKHKLEQLRNSAA |
| SEQ ID NO: 10 | MTEENVKRRTHNVLERQRRNELKRSFFALRDQIPELENNEKAPKVVILKKAT AYILSVQAETQKLISEIDLLRKQNEQLKHKLEQLRNSAA |

Thus, in a preferred embodiment, the functionally equivalent variant of the polypeptide of SEQ ID NO: 1 is selected from the group consisting of SEQ ID NO: 4, SEQ ID NO: 5, SEQ ID NO: 6, SEQ ID NO: 7, SEQ ID NO: 8, SEQ ID NO: 9 and SEQ ID NO: 10.

Additionally, functionally equivalent variants of Omomyc are also capable of transducing cells after the variant is contacted with said cell. It will be understood that functionally equivalent variants of Omomyc contain the protein transducing domain found in native Omomyc or another functional protein transducing domain.

In a preferred embodiment, a polypeptide is considered as a functionally equivalent variant of SEQ ID NO: 1 if it is capable of transducing a target cell at least 10%, 20%, 30%, 40%, 50%, 60%, 70%, 80%, 90% or 100% as efficiently as SEQ ID NO: 1.

Additionally, functionally equivalent variants of SEQ ID NO: 1 are also capable of translocating to the nucleus of the target cell.

In a preferred embodiment, a polypeptide is considered as a functionally equivalent variant of SEQ ID NO: 1 if it is capable of translocating to the nucleus of the target cells at least 10%, 20%, 30%, 40%, 50%, 60%, 70%, 80%, 90% or 100% as efficiently as the SEQ ID NO: 1.

Suitable assays for determining whether a polypeptide is a functionally equivalent variant of SEQ ID NO: 1 in terms of its ability to translocate across the cellular membrane and to the nucleus include double labelling of a cell with a reagent specific for the polypeptide and with a dye which specifically labels the nucleus of the cell (such as DAPI or Hoechst dye). The detection of the polypeptide for use according to the invention can be performed by confocal microscopy or by fluorescence microscopy.

In another preferred embodiment, the compound for use according to the invention is (b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof.

The term "conjugate", as used herein, refers to two or more compounds which are covalently linked together so that the function of each compound is retained in the conjugate.

The term "chemical moiety" refers to any chemical compound containing at least one carbon atom. Examples of chemical moieties include, but are not limited to, any peptide chain enriched in hydrophobic amino acids and hydrophobic chemical moieties.

In preferred embodiments, the conjugate for use according to the invention comprises at least 1, at least 2, at least 3, at least 4, at least 5, at least 6, at least 7, at least 8, at least 9, at least 10 or more chemical moieties that facilitate cellular uptake of the polypeptide or of the functionally equivalent variant of said polypeptide.

In one embodiment, the chemical moiety that facilitates cellular uptake of the polypeptide is a lipid or a fatty acid.

A fatty acid generally is a molecule comprising a carbon chain with an acidic moiety (e.g., carboxylic acid) at an end of the chain. The carbon chain of a fatty acid may be of any length, however, it is preferred that the length of the carbon chain be of at least 2, 3, 4, 5, 6, 7, 8, 9, 10, 11, 12, 13, 14, 15, 16, 17, 18, 19, 20 or more carbon atoms, and any range derivable therein. In certain embodiments, the length of the carbon chain is from 4 to 18 carbon atoms in the chain portion of the fatty acid. In certain embodiments the fatty acid carbon chain may comprise an odd number of carbon atoms, however, an even number of carbon atoms in the chain may be preferred in certain embodiments. A fatty acid comprising only single bonds in its carbon chain is called saturated, while a fatty acid comprising at least one double bond in its chain is called unsaturated. The fatty acid may be branched, though in preferable embodiments of the present invention, it is unbranched. Specific fatty acids include, but are not limited to, linoleic acid, oleic acid, palmitic acid, linolenic acid, stearic acid, lauric acid, myristic acid, arachidic acid, palmitoleic acid, arachidonic acid.

In a preferred embodiment, the chemical moiety that facilitates the cellular uptake of the polypeptide comprising the sequence SEQ ID NO: 1 or a functionally equivalent variant thereof, is a cell penetrating peptide sequence, in which case, the conjugate would comprise a fusion protein comprising the polypeptide comprising SEQ ID NO: 1 or the functionally equivalent variant thereof and the cell penetrating peptide sequence.

The term "fusion protein" relates to proteins generated by gene technology which consist of two or more functional domains derived from different proteins. A fusion protein may be obtained by conventional means, e.g., by means of gene expression of the nucleotide sequence encoding for said fusion protein in a suitable cell. It will be understood that the cell penetrating peptide refers to a cell penetrating peptide which is different from the cell penetrating peptide which forms part of the polypeptide comprising SEQ ID NO: 1 or of the functionally equivalent variant of SEQ ID NO: 1.

The term "cell penetrating peptide sequence" is used in the present invention interchangeably with "CPP", "protein transducing domain" or "PTD". It refers to a peptide chain of variable length that directs the transport of a protein inside a cell. The delivering process into cell commonly occurs by endocytosis but the peptide can also be internalized into cell by means of direct membrane translocation. CPPs typically have an amino acid composition that either contains a high relative abundance of positively charged amino acids such as lysine or arginine or has sequences that contain an alternating pattern of polar/charged amino acid and non-polar, hydrophobic amino acids.

Examples of CPPs which can be used in the present invention include, without limitation, the CPP found in Drosophila antennapedia protein (RQIKIWFQNRRMKWKK. SEQ ID NO:13) , the CPP found in the herpesvirus simplex 1 (HSV-1) VP22 DNA-binding protein (DAATATRGRSAASRPTERPRAPARSASRPRRPVE, SEQ ID NO:14) , the CPP of Bac-7 (RRIRPRPPRLPRPRPRPLPFPRPG; SEQ ID NO: 15), the CPPs of the HIV-1 TAT protein consisting of amino acids 49-57 (RKKRRQRRR, SEQ ID NO: 16), amino acids 48-60 (GRKKRRQRRRTPQ, SEQ ID NO: 17), amino acids 47-57 (YGRKKRRQRRR; SEQ ID NO: 18); the CPP of S413-PV peptide (ALWKTLLKKVLKAPKKKRKV; SEQ ID NO: 19), the CPP of penetratin (RQIKWFQNRRMKWKK; SEQ ID NO: 20), the CPP of SynB1 (RGGRLSYSRRRFSTSTGR; SEQ ID NO: 21), the CPP of SynB3 (RRLSYSRRRF; SEQ ID NO: 22), the CPP of PTD-4 (PIRRRKKLRRLK; SEQ ID NO: 23), the CPP of PTD-5 (RRQRRTSKLMKR; SEQ ID NO: 24), the CPP of the FHV Coat-(35-49) (RRRRNRTRRNRRRVR; SEQ ID NO: 25), the CPP of BMV Gag-(7-25) (KMTRAQRRAAARRNRWTAR; SEQ ID NO: 26), the CPP of HTLV-II Rex-(4-16) (TRRQRTRRARRNR; SEQ ID NO: 27), the CPP of D-Tat (GRKKRRQRRRPPQ; SEQ ID NO:28), the CPP R9-Tat (GRRRRRRRRRPPQ; SEQ ID NO: 29), the CPP of MAP (KLALKLALKLALALKLA; SEQ ID NO: 30), the CPP of SBP (MGLGLHLLVLAAALQGAWSQPKKKRKV; SEQ ID NO: 31), the CPP of FBP (GALFLGWLGAAGSTMGAWSQPKKKRKV; SEQ ID NO: 32), the CPP of MPG (ac-GALFLGFLGAAGSTMGAWSQPKKKRKV-cya; SEQ ID NO: 33), the CPP of MPG(ENLS) (ac-GALFLGFLGAAGSTMGAWSQPKSKRKV-cya; SEQ ID NO: 34), the CPP of Pep-1 (ac-KETWWETWWTEWSQPKKKRKV-cya; SEQ ID NO: 35), the CPP of Pep-2 (ac-KETWFETWFTEWSQPKKKRKV-cya; SEQ ID NO: 36), a polyarginine sequence having the structure RN (wherein N is between 4 and 17), the GRKKRRQRRR sequence (SEQ ID NO: 37), the RRRRRRLR sequence (SEQ ID NO: 38), the RRQRRTS KLMKR sequence (SEQ ID NO: 39); Transportan GWTLNSAGYLLGKINLKALAALAKKIL (SEQ ID NO: 40); KALAWEAKLAKALAKALAKHLAKALAKALKCEA (SEQ ID NO: 41); RQIKIWFQNRRMKWKK (SEQ ID NO: 42), the YGRKKRRQRRR sequence (SEQ ID NO: 43); the RKKRRQRR sequence (SEQ ID NO: 44); the YARAAARQARA sequence (SEQ ID NO: 45); the THRLPRRRRRR sequence (SEQ ID NO: 46); the GGRRARRRRRR sequence (SEQ ID NO: 47).

In a preferred embodiment, said cell-penetrating peptide is not the endogenous contained in SEQ ID NO: 1.

In a preferred embodiment, the cell-penetrating peptide sequence is selected from the group consisting of SEQ ID NO: 15, SEQ ID NO: 16, SEQ ID NO: 20, SEQ ID NO: 21, SEQ ID NO: 22, SEQ ID NO: 37 and SEQ ID NO: 38.

In another preferred embodiment, the CPP is the CPP of the HIV-1 TAT protein consisting of amino acids 49-57 (RKKRRQRRR, SEQ ID NO: 16). In another preferred embodiment the CPP is the GRKKRRQRRR sequence (SEQ ID NO: 37) or RRRRRRLR (SEQ ID NO: 38). In another embodiment, the CPP is the GRKKRRQRRR sequence (SEQ ID NO: 37) or RRRRRRRR (SEQ ID NO: 65).

In some embodiments, a CPP is as a CPP as described in WO2019/018898, the content of which is incorporated herein by reference in its entirety.

In one embodiment, the cell-penetrating peptide sequence is fused at the N-terminus of the polypeptide of the invention or of the functionally equivalent variant of said polypeptide. In another embodiment, the cell-penetrating peptide is fused at the C-terminus of the polypeptide of the invention or of the functionally equivalent variant of said polypeptide.

In preferred embodiments, the conjugates or fusion proteins of the compound for use according to the invention comprise, in addition to the own cell penetrating peptide found in the polypeptide of SEQ ID NO: 1 or of the functionally equivalent variant of said polypeptide, at least 1, at least 2, at least 3, at least 4, at least 5, at least 6, at least 7, at least 8, at least 9, at least 10 or more additional cell penetrating peptides.

Suitable fusion proteins of the invention include the polypeptides Omomyc*TAT and Omomyc*LZArg as defined below:

| Name | SEQ ID NO: | Sequence |
|---|---|---|
| Omomyc*TAT | 11 | MTEENVKRRTHNVLERQRRNELKRSFFALRDQIPELENNEKA PKVVILKKATAYILSVQAETQKLISEIDLLRKQNEQLKHKLEQLR NSCAGRKKRRQRRR |
| Omomyc*LZArg | 12 | MTEENVKRRTHNVLERQRRNELKRSFFALRDQIPELENNEKA PKVVILKKATAYILSVQAETQKLISEIDLLRKQNEQLKHKLEQLR NSCARRRRRLR |

Thus, is a preferred embodiment, the fusion protein is the polypeptide selected from SEQ ID NO: 11 and 12.

Suitable assays for determining whether a conjugate preserves the cell membrane translocation capacity of Omomyc include, without limitation, assays which measure the capacity of the conjugate to transduce cells in culture. This assay is based on contacting the conjugate with culture cells and detecting the presence of the conjugate in an intracellular location.

In another preferred embodiment, the conjugate of the compound for use according to the invention additionally comprises a further nuclear localization signal.

The term "nuclear localization signal" (NLS), as used herein, refers to an amino acid sequence of about 4-20 amino acid residues in length, which serves to direct a protein to the nucleus. Typically, the nuclear localization sequence is rich in basic amino acids and exemplary sequences are well known in the art (Gorlich D. (1998) EMBO 5.17:2721-7). In some embodiments, the NLS is selected from the group consisting of the SV40 large T Antigen NLS (PKKKRKV, SEQ ID NO: 48); the Nucleoplasmin NLS (KRPAATKKAGQAKKKK, SEQ ID NO: 49); the CBP80 NLS (RRRHSDENDGGQPHKRRK, SEQ ID NO: 50); the HIV-I Rev protein NLS (RQARRNRRRWE, SEQ ID NO: 51); the HTLV-I Rex (MPKTRRRPRRSQRKRPPT, SEQ ID NO: 52); the hnRNP A NLS (NQSSNFGPMKGGNFGGRSSGPYGGGGQYFKPRNQGGY, SEQ ID NO: 53); the rpL23a NLS (VHSHKKKKIRTSPTFTTPKTLRLRRQPKYPRKSAPRRNKLDHY, SEQ ID NO: 54). In one embodiment of the invention, the nuclear localization signal comprises the motif K (K/ R) X (K/ R) (SEQ ID NO: 55).

In an even more preferred embodiment, the nuclear localization signal is selected from the group consisting of PKKKRKV (SEQ ID NO: 48), PAAKRVKLD (SEQ ID NO: 56) and KRPAATKKAGQAKKKK (SEQ ID NO: 49).

In another preferred embodiment, the NLS may be N-terminal or C-terminal to the conjugate or the fusion protein comprising the polypeptide of SEQ ID NO: 1 or a functionally equivalent variant thereof.

The skilled person will understand that it may be desirable that the conjugate for use according to the invention further comprises one or more flexible peptides that connect the polypeptide comprising SEQ ID NO: 1 or the functionally equivalent variant thereof, the cell penetrating peptide sequence and/or the NLS. Thus, in a particular embodiment the polypeptide comprising SEQ ID NO: 1 or a functionally equivalent variant thereof is directly connected to the cell penetrating peptide sequence. In another particular embodiment, the polypeptide comprising SEQ ID NO: 1 or a functionally equivalent variant thereof is connected to the cell penetrating peptide sequence through a flexible peptide. In an embodiment the polypeptide comprising SEQ ID NO: 1 or the functionally equivalent variant thereof is directly connected to the NLS. In another embodiment the polypeptide comprising SEQ ID NO: 1 or a functionally equivalent variant thereof is connected to the NLS through a flexible peptide.

In a particular embodiment, the polypeptide of the conjugate for use according to the invention is directly connected to the cell penetrating peptide sequence and to the NLS.

In one embodiment, the NLS is one of the NLS which appears endogenously in the Myc sequence, such as the M1 peptide (PAAKRVKLD, SEQ ID NO: 56) or the M2 peptide (RQRRNELKRSF, SEQ ID NO: 57).

In another embodiment the additional NLS refers to a NLS which is different to the endogenous NLS found in polypeptide comprising SEQ ID NO: 1 or in the functionally equivalent variant of SEQ ID NO: 1.

In preferred embodiments, the conjugates or fusion proteins for use according to the invention comprise, in addition to the endogenous NLS found in the polypeptide of the invention or in the functionally equivalent variant thereof, at least 1, at least 2, at least 3, at least 4, at least 5, at least 6, at least 7, at least 8, at least 9, at least 10 NLS.

In another particular embodiment, the polypeptide of the conjugate for use according to the invention is connected to the cell penetrating peptide sequence through a first flexible peptide linker and to the NLS through a second flexible peptide linker.

As used herein, the term "flexible peptide", "spacer peptide" or "linker peptide" refers to a peptide that covalently binds two proteins or moieties but which is not part of either polypeptide, allowing movement of one with respect to the other, without causing a substantial detrimental effect on the function of either the protein or the moiety. Thus, the flexible linker does not affect the activity of the polypeptide sequence, the cell penetrating activity of the cell penetrating peptide or the nuclear localization capacity of the NLS.

The flexible peptide comprises at least one amino acid, at least two amino acids, at least three amino acids, at least four amino acids, at least five amino acids, at least six amino acids, at least seven amino acids, at least eight amino acids, at least nine amino acids, at least 10 amino acids, at least 12 amino acids, at least 14 amino acids, at least 16 amino acids, at least 18 amino acids, at least 20 amino acids, at least 25 amino acids, at least 30 amino acids, at least 35 amino acids, at least 40 amino acids, at least 45 amino acids, at least 50 amino acids, at least 60 amino acids, at least 70 amino acids, at least 80 amino acids, at least 90 amino acids, or about 100 amino acids. In some embodiments the flexible peptide will permit the movement of one protein with respect to the other in order to increase solubility of the protein and/or to improve its activity. Suitable linker regions include a poly-glycine region, the GPRRRR sequence (SEQ ID NO: 58) of combinations of glycine, proline and alanine residues.

In a particular embodiment, the conjugates for use according to the invention comprise a tag bound to the conjugate or to the C-terminal or N-terminal domain of said polypeptide or fusion protein or variant thereof. Said tag is generally a peptide or amino acid sequence which can be used in the isolation or purification of said fusion protein. Thus, said tag is capable of binding to one or more ligands, for example, one or more ligands of an affinity matrix such as a chromatography support or bead with high affinity. An example of said tag is a histidine tag (His-tag or HT), such as a tag comprising 6 residues of histidine (His6 or H6), which can bind to a column of nickel ($Ni^{2+}$) or cobalt ($Co^{2+}$) with high affinity. His-tag has the desirable feature that it can bind its ligands under conditions that are denaturing to most proteins and disruptive to most protein-protein interactions. Thus, it can be used to remove the bait protein tagged with H6 following the disruption of protein-protein interactions with which the bait has participated.

Additional illustrative, non-limitative, examples of tags useful for isolating or purifying the conjugate or the polypeptide comprising SEQ ID NO: 1 or a variant thereof or a fusion protein include Arg-tag, FLAG-tag (DYKDDDDK; SEQ ID NO: 59), Strep-tag (WSHPQFEK, SEQ ID NO:60), an epitope capable of being recognized by an antibody, such as c-myc-tag (recognized by an anti-c-myc antibody), HA tag (YPYDVPDYA, SEQ ID NO:61), V5 tag (GKPIPNPLLGLDST, SEQ ID NO:62), SBP-tag, S-tag, calmodulin binding peptide, cellulose binding domain, chitin binding domain, glutathione S-transferase-tag, maltose binding protein, NusA, TrxA, DsbA, Avi-tag, etc. (Terpe K., Appl. Microbiol. Biotechnol. 2003, 60:523-525), an amino acid sequence such as AHGHRP (SEQ ID NO: 63) or PIHDHDHPHLVIHSGMTCXXC (SEQ ID NO: 64), β-galactosidase and the like.

The tag can be used, if desired, for the isolation or purification of said fusion protein.

In another preferred embodiment, the compound for use according to the invention is (c) a polynucleotide, hereinafter the polynucleotide of the invention, encoding the polypeptide or the fusion protein disclosed above. In a preferred embodiment, the compound for use according to the invention is a polynucleotide encoding a polypeptide comprising the sequence SEQ ID NO: 1 or a functionally equivalent variant thereof. In another embodiment, the compound for use according to the invention is a polynucleotide encoding a conjugate comprising the polypeptide comprising the sequence SEQ ID NO: 1 or a functionally equivalent variant thereof and a chemical moiety that facilitates cellular uptake of the polypeptide or of the functionally equivalent variant thereof; more preferably is a polynucleotide encoding a fusion protein between the polypeptide comprising the sequence SEQ ID NO: 1 or a functionally equivalent variant thereof and a cell-penetrating peptide sequence.

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a cell.

In a preferred embodiment, the polynucleotide for use according to the invention is an mRNA. mRNA can be chemically synthesized, can be obtained by means of *in vitro* transcription or can be synthesized *in vivo* in the target cell. The nucleotide sequences that form the polynucleotide encoding the conjugate or fusion protein for use according to the invention are in the same correct reading frame for expression thereof.

In a preferred embodiment, the compound for use according to the invention is a mRNA encoding for a polypeptide consisting of the sequence SEQ ID NO: 1 or a polypeptide consisting of a functionally equivalent variant of SEQ ID NO: 1 or a polypeptide consisting of SEQ ID NO: 4.

In another embodiment, the compound for use according to the invention is (d) a vector comprising the polynucleotide of the invention.

The term "vector", as used herein, refers to a nucleic acid sequence comprising the necessary sequences so that after transcribing and translating said sequences in a cell, a polypeptide encoded by the polynucleotide of the invention is generated. Said sequence is operably linked to additional segments that provide for its autonomous replication in a host cell of interest. Preferably, the vector is an expression vector, which is defined as a vector which, in addition to the regions of the autonomous replication in a host cell, contains regions operably linked to the nucleic acid of the invention and which are capable of enhancing the expression of the products of the nucleic acid according to the invention. The vectors for use according to the invention can be obtained by means of techniques widely known in the art.

Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. Suitable vectors comprising a polynucleotide of the invention are vectors derived from expression vectors in prokaryotes such as pUC18, pUC19, pBluescript and their derivatives, mp18, mp19, pBR322, pMB9, ColE1, pCRI, RP4, phages and "shuttle" vectors such as pSA3 and pAT28, expression vectors in yeasts such as vectors of the 2-micron plasmid type, integration plasmids, YEP vectors, centromeric plasmids and similar, expression vectors in insect cells such as the vectors of the pAC series and of the pVL series, expression vectors in plants such as vectors of the series pIBl, pEarleyGate, pAVA, pCAMBIA, pGSA, pGWB, pMDC, pMY, pORE and similar and expression vectors in superior eukaryote cells based on viral vectors (adenovirus, virus associated to adenovirus as well as retrovirus and, in particular, lentivirus) as well as non-viral vectors such as pSilencer 4.1-CMV (Ambion), pcDNA3, pcDNA3.1/hyg, pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL, pKSV-10, pBPV-1, pML2d and pTDT1. In a preferred embodiment, the polynucleotide for use according to the invention is comprised in a vector selected from the group consisting of pEGFP or pBabe retroviral vectors and pTRIPZ or pSLIK lentiviral vectors.

The vector for use according to the invention may be used to transform, transfect or infect cells that can be transformed, transfected or infected by said vector. Said cells may be prokaryotic or eukaryotic.

The vector preferably comprises the polynucleotide for use according to the invention operationally bound to sequences that regulate the expression of the polynucleotide. The regulatory sequences of use in the present invention may be nuclear promoters or, alternatively, enhancer sequences and/or other regulatory sequences that increase expression of the heterologous nucleic acid sequence. In principle, any promoter can be used in the present invention provided said promoter is compatible with the cells wherein the polynucleotide is to be expressed. Thus, promoters suitable for realizing the present invention include, but are not necessarily limited to, constitutive promoters such as derivatives of eukaryotic virus genomes such as polyoma virus, adenovirus, SV40, CMV, avian sarcoma virus, hepatitis B virus, the metallothionein gene promoter, the herpes simplex virus thymidine kinase gene promoter, LTR regions of retroviruses, the immunoglobulin gene promoter, the actin gene promoter, the EF-1alpha gene promoter as well as inducible promoters wherein protein expression depends on the addition of a molecule or exogenous signal, such as tetracycline systems, the NFκB/UV light system, the Cre/Lox system and the heat shock genes promoter, the regulable RNA polymerase II promoters described in WO/2006/135436 and tissue-specific promoters.

In another embodiment, the compound for use according to the invention is (e) a cell capable of secreting into the medium the polypeptide of the invention or the conjugate of the invention, preferably the polypeptide of the invention or the fusion protein of the invention.

Suitable cells capable of secreting a polypeptide of the invention include without limitation, cardiomyocytes, adipocytes, endothelial cells, epithelial cells, lymphocytes (B and T cells), mastocytes, eosinophils, vascular intima cells, primary cultures of isolated cells of different organs, preferably of cells isolated from Langerhans islets, hepatocytes, leukocytes, including mononuclear leukocytes, mesenchymal, umbilical cord or adult (of skin, lung, kidney and liver), osteoclasts, chondrocytes and other connective tissue cells.

Cells of established lines such as Jurkat T cells, NIH-3T3, CHO, Cos, VERO, BHK, HeLa, COS, MDCK, 293, 3T3 cells, C2C12 myoblasts and W138 cells are also suitable. Persons skilled in the art will appreciate that the cells capable of secreting into the medium a polypeptide of the invention may be found forming microparticles or microcapsules so that the cells have a greater useful life in patients. Materials suitable for the formation of microparticles object of the invention include any biocompatible polymeric material which permits continuous secretion of the therapeutic products and which acts as support of the cells. Thus, said biocompatible polymeric material may be, for example, thermoplastic polymers or hydrogen polymers. Examples of thermoplastic polymers are acrylic acid, acrylamide, 2-aminoethyl methacrylate, poly(tetrafluoroethylene-cohexafluorpropylene), methacrylic-(7-cumaroxy) ethyl ester acid, N-isopropyl acrylamide, polyacrylic acid, polyacrylamide, polyamidoamine, poly(amino)-p-xylylene, poly(chloroethylvinylether), polycaprolactone, poly(caprolactone-co-trimethylene carbonate), polycarbonate urea) urethane, poly(carbonate) urethane, polyethylene, polyethylene and acrylamide copolymer, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic glycolic acid), poly(L-lactic acid), poly(gamma-methyl, L-glutamate), poly(methylmethacrylate), polypropylene fumarate), polypropylene oxide), polypyrrole, polystyrene, poly(tetrafluoroethylene), polyurethane, polyvinyl alcohol, polyethylene of ultra-high molecular weight, 6-(p-vinylbenzamide)-hexanoic acid N-p-vinylbenzyl-D-maltonamide and copolymers containing more than one of said polymers. Examples of polymers of hydrogel type are natural materials of alginate, agarose, collagen, starch, hyaluronic acid, bovine serum albumin, cellulose and their derivatives, pectin, chondroitin sulphate, fibrin and fibroin, as well as synthetic hydrogels such as Sepharose® and Sephadex®.

The compound for use according to the invention is for use in the prevention and/or treatment of an autoimmune disease in a subject.

Thus, in an aspect, the present invention relates to a compound selected from the group consisting of:

(a) a polypeptide comprising the sequence SEQ ID NO: 1 or a functionally equivalent variant thereof,

(b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,

(c) a polynucleotide encoding the polypeptide of a) or the conjugate of b),

(d) a vector comprising the polynucleotide according to c), and

(e) a cell capable of secreting into the medium the polypeptide according to a) or the conjugate according to b)

for use in the prevention of an autoimmune disease in a subject.

The term "prevention" is understood as the administration of the compound for use according to the invention in an initial or early stage of the disease, or to also prevent its onset. In a particular embodiment, the term "prevention" refers to the administration of the compound prior to the manifestation of the autoimmune disease, i.e. before symptoms manifest.

In another aspect, the present invention relates to a compound selected from the group consisting of:

(a) a polypeptide comprising the sequence SEQ ID NO: 1 or a functionally equivalent variant thereof,

(b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,

(c) a polynucleotide encoding the polypeptide of a) or the conjugate of b),

(d) a vector comprising the polynucleotide according to c), and

(e) a cell capable of secreting into the medium the polypeptide according to a) or the conjugate according to b)

for use in the treatment of an autoimmune disease in a subject.

The term "treatment" is used to designate the administration of the compound for use according to the invention to control the progression of the disease before or after the clinical signs have appeared. Control of the progression of the disease is understood as the beneficial or desired clinical results which include but are not limited to: reduction of the symptoms, reduction of the duration of the disease, stabilization of pathological conditions (specifically avoiding additional impairment), delaying the progression of the disease, improving the pathological condition and remission (both partial and complete). The control of the progression of the disease also involves a prolongation of survival in comparison to the expected survival if the treatment was not applied. In a preferred embodiment, the control of the progression of the disease is measured as reduction in loss of weight. In another preferred embodiment, the control of the progression of the disease is measured as a lower clinical score, preferably measured as in the examples of the present invention. In another preferred embodiment, the control of the progression of the disease is measured as a lower neurological disability score, preferably measured as in the examples of the present invention. In another embodiment, the control of the progression of the disease is measured as a delay in the day of disease onset.

The term "autoimmune disease", is used in the present invention to refer to a pathogenic condition in which the patient's immune system results in disease from a self-antigen (autoimmunity). Autoimmune diseases form a group of diseases caused by an impairment of the immune system that makes it react against the body's own tissues. Autoimmunity is present in everyone to some extent. It is usually harmless and probably a universal phenomenon of vertebrate life. However, autoimmunity can be the cause of a broad spectrum of human illnesses, known as autoimmune diseases. The modification of the cell recognition mechanisms that normally enable the body to distinguish between the "self" and the "non-self", i.e., between elements that belong to the body and elements that are foreign to it, give rise to the production of antibodies and differentiation of specific T and B cells, which can target single organs (organ-specific diseases) or trigger systemic disorders, damaging the individual's functions as a whole. Autoimmune diseases are, thus, defined when the progression from benign autoimmunity to pathogenic autoimmunity occurs. This progression is determined by both genetic influences and environmental triggers. Autoimmune diseases, which may be treated and/or prevented by the present invention include systemic lupus erythematosus (SLE), lupus nephritis, central nervous system (CNS) lupus, diabetes mellitus (type I), asthma, ulcerative colitis, Crohn's disease, Grave's disease, Addison's disease, celiac disease, alopecia areata, arthritis, including rheumatoid arthritis and osteoarthritis, pernicious anemia, Latent Autoimmune Diabetes in Adults (LADA), and multiple sclerosis, among numerous others.

Numerous autoimmune diseases may be treated using the compound for use according to the invention, including autoimmune blood diseases, including pernicious anemia, autoimmune hemolytic anemia, aplastic anemia, idiopathic thrombocytopenic purpura, ankylosing spondilitis; autoimmune diseases of the musculature including polymyositis and dermatomyositis; autoimmune diseases of the ear including autoimmune hearing loss and Meniere's syndrome; autoimmune eye diseases, including Mooren's disease, Reiter's syndrome, Vogt-Koyanagi-Harada disease and acute anterior uveitis; autoimmune diseases of the kidney including glomerulonephritis, IgA nephropathy, and lupus nephritis; diabetes mellitus (type I); autoimmune skin diseases including pemphigus (autoimmune bullous diseases), such as pemphigus vulgaris, pemphigus foliaceus, pemphigus erythematosus, bullous pemphigoid, vitiligo, epidermolysis bullosa acquisita, psoriasis and alopecia areata; cardiovascular autoimmune diseases, including autoimmune myocarditis, vasculitis including Churg-Strauss syndrome, giant cells arteritis, Kawasaki's disease, polyarteritis nodosa, Takayasu's arteritis and Wegener's granulomatosis; endocrine autoimmune diseases, including Addison's disease, autoimmune hypoparathyroidism, autoimmune hypophysitis, autoimmune oophoritis, autoimmune orchitis, Grave's Disease, Hashimoto's thyroiditis, polyglandular autoimmune syndrome type 1 (PAS-I), polyglandular autoimmune syndrome type 2 (PAS-2), and polyglandular autoimmune syndrome type 3 (PAS-3); autoimmune gastroenteric diseases including autoimmune hepatitis, primary biliary cirrhosis, inflammatory bowel disease, celiac disease, Crohn's disease; autoimmune nervous diseases, including multiple sclerosis, myasthenia gravis, Guillain-Barre syndrome and chronic inflammatory demyelinating neuropathy; and systemic autoimmune diseases including systemic lupus erythematosus, antiphospholid syndrome, autoimmune lymphoproliferative disease, autoimmune polyendocrinopathy, Bechet's disease, Goodpasture's disease, arthrtitis, including rheumatoid arthritis, osteoarthritis and septic arthritis, sarcoidosis, scleroderma and Sjogren's syndrome and psoriasis among others.

In a particular embodiment, the autoimmune disease is selected from the group consisting of: celiac disease, diabetes mellitus (type I), Graves' disease, inflammatory bowel disease, multiple sclerosis, alopecia areata, Addison's disease, pernicious anemia, psoriasis, rheumatoid arthritis, Latent Autoimmune Diabetes in Adults (LADA), and systemic lupus erythematosus.

In a more particular embodiment, the autoimmune disease is selected from the group consisting of diabetes mellitus (type I), multiple sclerosis and psoriasis.

In a preferred embodiment, the autoimmune disease is a central nervous system (CNS) autoimmune demyelinating disease.

A CNS autoimmune demyelinating disease is a disease wherein myelin-supporting cells of the CNS, such as oligodendrocytes, and/or the myelin lamellae are destroyed. Demyelination leads to a disruption in neural signals between the brain and other parts of the body, ultimately resulting in a range of signs and symptoms, including physical, mental, and sometimes psychiatric problems.

The CNS autoimmune demyelinating diseases that can be treated by the present invention are, without limitation, multiple sclerosis (MS), clinically isolated syndrome (CIS), tumefactive (tumor-like) MS, Marburg's acute MS, Balós's concentric sclerosis, acute disseminated encephalomyelitis (ADEM), acute hemorrhagic leukoencephalitis, Schilder's disease, solitary sclerosis, transverse myelitis, Susac's syndrome, leukoaraiosis, myalgic encephalomyelitis, Guillain-Barré syndrome, progressive inflammatory neurophathy, leukodystrophy, including adrenoleukodystrophy, adrenomyeloneuropathy, post-vaccinal encephalitis (PVE), post-infectious encephalomyelitis (PIE), neuromyelitis optica (NMO), optic neuritis and myelin oligodendrocyte glycoprotein (MOG)-associated disease (MOGAD). Preferably, the CNS autoimmune demyelinating disease is selected from the group consisting of multiple sclerosis (MS), clinically isolated syndrome (CIS), tumefactive (tumor-like) MS, Marburg's acute MS, Balós's concentric sclerosis, acute disseminated encephalomyelitis (ADEM), post-vaccinal encephalitis (PVE), post-infectious encephalomyelitis (PIE) and neuromyelitis optica (NMO). In a more particular embodiment the autoimmune disease is selected from multiple sclerosis (MS), acute disseminated encephalomyelitis (ADEM) and post-vaccinal encephalitis (PVE). In a still more preferred embodiment, the autoimmune disease is multiple sclerosis.

There is a lack of a natural non-human counterpart for MS. Therefore, EAE model is an accepted animal model of MS and other CNS autoimmune demyelinating diseases characterized by demyelination and neurological dysfunction along with corresponding clinical symptoms (Baxter AG. The origin and application of experimental autoimmune encephalomyelitis. Nat Rev Immunol 2007, 7:904-912; Storch MK et al. Autoimmunity to myelin oligodendrocyte glycoprotein in rats mimics the spectrum of multiple sclerosis pathology. Brain Pathol. 1998, 8(4):681-94 and Hirano Y. Acute disseminated encephalomyelitis. Nippon Rinsho. 1997, 55(4):934-9.).

The experiments of the present invention were performed in the experimental autoimmune encephalomyelitis model, sometimes called experimental allergic encephalomyelitis (EAE) model. EAE is an inflammatory demyelinating disease of the CNS that acts as an animal model of brain inflammation. It is mostly used with rodents and is widely studied as an animal model of the human CNS demyelinating diseases, including multiple sclerosis (MS), clinically isolated syndrome (CIS), in particular for the treatment of multiple sclerosis.

Multiple sclerosis has several forms of presentation, including episodic acute period of worsening (the most common, initially), gradual progressive deterioration of neurologic functions or combinations of both. These different presentations have been given standardized names, which are relapsing-remitting (RR), secondary progressive (SP), primary progressive (PP), and progressive relapsing (PR). RR-MS is defined as clearly defined disease relapses with either full recovery or sequelae and residual deficit upon recovery; period between disease relapses are characterized by a lack of disease progression. Approximately 85% of patients start out with RR-MS (Lublin FD et al., Defining the clinical course of multiple sclerosis: the 2013 revisions. Neurology. 2014 Jul 15;83(3):278-86).

The term "subject", as used herein, includes any animal that has an autoimmune disease or exhibits a symptom of an autoimmune disease, or is at risk for having an autoimmune disease or exhibiting a symptom of autoimmune disease. Suitable subjects (patients) include laboratory animals (such as mouse, rat, rabbit, or guinea pig), farm animals, and domestic animals or pets (such as cats or dogs). Non-human primates and, preferably, human patients, are included. Preferably, the subject is a mammal.

The term "mammal" includes, but is not restricted to, domestic and farm mammals, primates and humans, e.g., human beings, non-human primates, cows, horses, pigs, sheep, goats, dogs, cats or rodents. In a preferred embodiment the mammal is a human.

The compound for use according to the invention can be incorporated into pharmaceutical compositions for its administration to the subject, wherein the pharmaceutical composition further comprises at least one pharmaceutically acceptable excipient.

The pharmaceutical composition comprises the compound for use according to the invention in a therapeutically effective amount.

Thus, in a particular embodiment of the first aspect of the invention, the compound for use according to the invention is comprised within a pharmaceutical composition wherein the pharmaceutical composition further comprises at least one pharmaceutically acceptable excipient.

In another aspect, the invention relates to a pharmaceutical composition comprising a compound according to the invention and a pharmaceutically acceptable excipient for use in the prevention and/or treatment of an autoimmune disease in a subject.

The term "pharmaceutically acceptable excipient" is understood a therapeutically inactive substance said to be used for incorporating the active ingredient and which is acceptable for the patient from a pharmacological/toxicological point of view and for the pharmaceutical chemist who manufactures it from a physical/chemical point of view with respect to the composition, formulation, stability, acceptation of the patient and bioavailability. The excipient can be a carrier. As used herein "carrier" is meant any substance that serves to improve the delivery and the effectiveness of the active principle within the pharmaceutical composition. In a preferred embodiment, the carrier does not allow direct delivery of the compound to the cytoplasm of the cells, i.e. the carrier is not capable of fusing with the plasmatic membrane of the target cells. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of components forming part of the compositions for use of the invention. Examples of proper carriers are well known in the literature (see for example Remington's Pharmaceutical Sciences, 19th ed., Mack Publishing Company, Easton, PA, 1995). Examples of carriers without limitation are a series of saccharide such as lactose, dextrose, sucrose, sorbitol, mannitol, xylitol, erythritol, and maltitol; a series of starch such as corn starch, wheat starch, rice starch, and potato starch; a series of cellulose such as cellulose, methyl cellulose, sodium carboxy methyl cellulose, and hydroxyl propylmethyl cellulose; and a series of filler such as gelatin and polyvinyl pyrrolidone. In some cases, a disintegrant such as cross-linked polyvinyl pyrrolidone, agar, alginic acid, or sodium alginate may be added.

The number and the nature of the pharmaceutically acceptable excipients depend on the desired dosage form. The pharmaceutically acceptable excipients are known by the person skilled in the art (Fauli y Trillo C. (1993) "Tratado de Farmacia Galénica", Luzán 5, S.A. Ediciones, Madrid). Said compositions can be prepared by means of the conventional methods known in the state of the art ("Remington: The Science and Practice of Pharmacy", 20th edition (2003) Genaro A.R., ed., Lippincott Williams & Wilkins, Philadelphia, US).

For pharmaceutical compositions comprising an agent that is a nucleic acid molecule, the nucleic acid molecule may be present within any of a variety of delivery systems known to those of ordinary skill in the art, including nucleic acid, and bacterial, viral and mammalian expression systems such as, for example, recombinant expression constructs as provided herein. Techniques for incorporating DNA into such expression systems are well known to those of ordinary skill in the art. The DNA may also be "naked," as described, for example, in Ulmer et al., Science 259:1745-49, 1993 and reviewed by Cohen, Science 259:1691-1692, 1993. The uptake of naked DNA may be increased by coating the DNA onto biodegradable beads, which are efficiently transported into the cells.

Nucleic acid molecules may be delivered into a cell according to any one of several methods described in the art (see, e.g., Akhtar et al., Trends Cell Bio. 2:139 (1992); Delivery Strategies for Antisense Oligonucleotide Therapeutics, ed. Akhtar, 1995, Maurer et al., Mol. Membr. Biol. 16:129-40 (1999); Hofland and Huang, Handb. Exp. Pharmacol. 137:165-92 (1999); Lee et al., ACS Symp. Ser. 752:184-92 (2000); U.S. Pat. No. 6,395,713; International Patent Application Publication No. WO 94/02595); Selbo et al., Int. J. Cancer 87:853-59 (2000); Selbo et al., Tumour Biol. 23:103-12 (2002); U.S. Patent Application Publication Nos. 2001/0007666, and 2003/077829). Such delivery methods known to persons having skill in the art, include, but are not restricted to, encapsulation in liposomes, by iontophoresis, or by incorporation into other vehicles, such as biodegradable polymers; hydrogels; cyclodextrins (see, e.g., Gonzalez et al., Bioconjug. Chem. 10: 1068-74 (1999); Wang et al., International Application Publication Nos. WO 03/47518 and WO 03/46185); poly (lactic-co-glycolic) acid (PLGA) and PLCA microspheres (also useful for delivery of peptides and polypeptides and other substances) (see, e.g., U.S. Pat. No. 6,447,796; U.S. Patent Application Publication No. 2002/130430); biodegradable nanocapsules; and bioadhesive microspheres, or by proteinaceous vectors (International Application Publication No. WO 00/53722). In another embodiment, the nucleic acid molecules can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives (see also, e.g., U.S. Patent Application Publication No. 2003/0077829).

In a particular embodiment, when the compound for use according to the invention is a nucleic acid, the pharmaceutical composition may be formulated as a composition intended for use in gene therapy; by way of illustration, not limitation, that pharmaceutical composition may contain a viral or non-viral vector, which comprises the suitable polynucleotide or gene construction. By way of illustration and not limitation, said vectors, may be viral, for example, based on retrovirus, adenovirus, etc., or non-viral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain the corresponding polynucleotide or gene construction, may be administered directly to a subject by conventional methods. Alternatively, said vectors may be used to transform, or transfect or infect cells, for example, mammal cells, including human, ex vivo, which subsequently will be implanted into a human body or an animal to obtain the desired therapeutic effect. For administration to a human body or an animal, said cells will be formulated in a suitable medium that will have no adverse influence on cell viability.

The pharmaceutical composition comprising the compound for use according to the present invention comprises the compound in a therapeutically effective amount.

The expression "therapeutically effective amount", as used herein, is understood as an amount capable of providing a therapeutic effect, and which can be determined by the person skilled in the art by commonly used means. The amount of the Omomyc polypeptide, of the functionally equivalent variant thereof, of the conjugate, fusion protein, polynucleotide, vector or cell vary depending upon the subject and the particular mode of administration. Those skilled in the art will appreciate that dosages may also be determined with guidance from Goodman and Goldman's The Pharmacological Basis of Therapeutics, Ninth Edition (1996), Appendix II, pp. 1707-1711 and from Goodman and Goldman's The Pharmacological Basis of Therapeutics, Tenth Edition (2001), Appendix II, pp. 475-493.

The appropriate dosage of the active principle or principles within the pharmaceutical composition will depend on the type of autoimmune disease to be treated, the severity and course of the disease, whether the composition is administered for preventive or therapeutic purposes, previous therapy, the patient's clinical history and response to the peptide or polypeptide, and the discretion of the attending physician.

Doses of the compound for use according to the invention may be expressed either in mg of compound per kg of body weight or in mg of compound per square meter of body surface. The article from Reagan-Shaw S. et al. (Reagan-Shaw S. et al. "Dose translation from animal to human studies revisited". FASEB J 2008, 22(3):659-661) provides the standard conversion factors used to convert mg/kg to mg/m$^2$.

$$\text{Dose (mg/kg)} \times K_m = \text{Dose (mg/m}^2)$$

The article also explains that this conversion is the basis for converting dose in a first animal species to dose in a second animal species (allometric dose translation). Thus, animal dose (AD) in mg/kg can be converted to human equivalent dose (HED) in mg/kg using the following formula:

$$\text{HED (mg/kg)} = \text{AD (mg/kg)} \times \frac{\text{Animal } K_m}{\text{Human } K_m}$$

wherein the $K_m$ for each species is shown in Table 1 (data extracted from Reagan-Shaw S. et al. "Dose translation from animal to human studies revisited". FASEB J 2008, 22(3):659-661).

**Table 1.** $K_m$ factor for conversion of AD to HED

| Species | | $K_m$ factor |
|---|---|---|
| Human | Adult | 37 |
| | Child | 25 |
| Baboon | | 20 |
| Dog | | 20 |
| Monkey | | 12 |
| Rabbit | | 12 |
| Guinea pig | | 8 |
| Rat | | 6 |
| Hamster | | 5 |
| Mouse | | 3 |

The amount of polypeptide comprising the sequence SEQ ID NO: 1, the functionally equivalent variant thereof, the fusion protein, the conjugate, polynucleotide, vector or cell is suitably administered to the patient at one time or over a series of treatments. Depending on the type and severity of the disease, an appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. Alternatively, the dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated.

In a particular embodiment, the compound or the pharmaceutical composition comprising the compound for use according to the invention is administered in a regimen comprising a plurality of administrations (i.e. at least two administrations). In a preferred embodiment, the regimen comprises at least 2 administrations, at least 3, at least 4, at least 5, at least 6, at least 7, at least 8, at least 9, at least 10. In a more particular embodiment, the regimen comprises at least 3 administrations. In a still more preferred embodiment, the regimen comprises 4 administrations.

In an embodiment, the compound is administered daily. In another embodiment, the compound is administered every other day.

In an embodiment, the compound can be administered once a week, twice a week, three times a week, four times a week, five times a week, six times a week or seven times a week. In an embodiment, the compound can be administered once a week. In another embodiment, the compound can be administered twice per week. In another embodiment, the compound can be administered three times a week.

The duration of the treatment can be at least 3 days, at least 4 days, at least 5 days, at least 6 days, at least one week, at least two weeks, at least three weeks, at least four weeks, at least five weeks, at least six weeks, at least seven weeks, at least eight weeks, at least nine weeks, at least ten weeks or more. Preferably, the duration of the treatment is at least 7 days.

In a particular embodiment, the compound for use or the pharmaceutical composition comprising the compound for use according to the invention is administered in the early stages of the autoimmune response.

In a particular embodiment, the compound for use according to the invention is administered at a dosage level between 0.1 and 100 mg/kg per day, preferably between 1 and 85 mg/kg per day, more preferably between 5 and 75 mg/kg per day.

In a particular embodiment, the amount of the compound comprising the sequence SEQ ID NO: 1, the functionally equivalent variant thereof, the fusion protein, the conjugate, polynucleotide, vector or cell, preferably the amount of the polypeptide or the functionally equivalent variant thereof, is of about 50 mg/kg, of subject body weight per day, preferably administered every day, preferably intraperitoneally administered, more preferably intravenously administered. In a preferred embodiment, the amount of the compound comprising the sequence SEQ ID NO: 1, the functionally equivalent variant thereof, the fusion protein, the conjugate, polynucleotide, vector or cell, preferably the amount of the polypeptide or the functionally equivalent variant thereof, is of about 75 to 200 mg/m², preferably of 100 to 175 mg/m², more preferably 150 mg/m², preferably administered every day, preferably intraperitoneally administered, more preferably intravenously administered.

In another particular embodiment, the amount of the compound comprising the sequence SEQ ID NO: 1, the functionally equivalent variant thereof, the fusion protein, the conjugate, polynucleotide, vector or cell, preferably the amount of the polypeptide or the functionally equivalent variant thereof, is of about 75 mg/kg, of subject body weight per day, preferably administered 3 days per week, preferably intravenously administered. In a preferred embodiment, the amount of the compound comprising the sequence SEQ ID NO: 1, the functionally equivalent variant thereof, the fusion protein, the conjugate, polynucleotide, vector or cell, preferably the amount of the polypeptide or the functionally equivalent variant thereof, is of about 75 to 275 mg/m², preferably of 100 to 250 mg/m², more preferably 225 mg/m², preferably administered 3 days per week, preferably intravenously administered.

In another particular embodiment, the amount of the compound comprising the sequence SEQ ID NO: 1, the functionally equivalent variant thereof, the fusion protein, the conjugate, polynucleotide, vector or cell, preferably the amount of the polypeptide or the functionally equivalent variant thereof, is of about 75 mg/kg, of subject body weight per day, preferably administered every other day, preferably administered in 4 doses, preferably intravenously administered. In a preferred embodiment, the amount of the compound comprising the sequence SEQ ID NO: 1, the functionally equivalent variant thereof, the fusion protein, the conjugate, polynucleotide, vector or cell, preferably the amount of the polypeptide or the functionally equivalent variant thereof, is of about 75 to 275 mg/m², preferably of 100 to 250 mg/m², more preferably 225 mg/m², preferably administered every other day, preferably administered in 4 doses, preferably intravenously administered.

In some embodiments, when the compound comprising the sequence SEQ ID NO: 1, the functionally equivalent variant thereof, the fusion protein, the conjugate, polynucleotide, vector or cell, preferably the polypeptide or the functionally equivalent variant thereof, is used for the prevention and/or treatment of a human patient, it will be administered in quantities ranging from 0.01 mg/kg/day to 8 mg/kg/day, preferably 0.5 mg/kg/day to 7.5 mg/kg/day, more preferably 1 mg/kg/day to 7 mg/kg/day, more preferably 1.5 mg/kg/day to 6.75 mg/kg/day, more preferably 2 mg/kg/day to 6.5 mg/kg/day, more preferably 3 mg/kg/day to 6.25 mg/kg/day, more preferably 4.05 mg/kg/day to 6 mg/kg/day. In a preferred embodiment, is administered daily, preferably is intraperitoneally administered, even more preferably is intravenously administered.

In some embodiments, when the compound comprising the sequence SEQ ID NO: 1, the functionally equivalent variant thereof, the fusion protein, the conjugate, polynucleotide, vector or cell, preferably the polypeptide or the functionally equivalent variant thereof, is used for the prevention and/or treatment of a human patient, it will be administered in quantities ranging from 50 to 500 mg/m², preferably 60 to 450 mg/m², more preferably 70 to 400 mg/m², more preferably 80 to 350 mg/m², more preferably 90 to 300 mg/m², more preferably 95 to 250 mg/m², more preferably 100 to 200 mg/m², more preferably 105 to 190 mg/m², more preferably 110 to 185 mg/m², more preferably 115 to 180 mg/m², more preferably 120 to 170 mg/m², more preferably 125 to 160 mg/m². In a preferred embodiment, is administered daily, preferably is administered intraperitoneally, more preferably is administered intravenously.

In another particular embodiment, when the compound comprising the sequence SEQ ID NO: 1, the functionally equivalent variant thereof, the fusion protein, the conjugate, polynucleotide, vector or cell, preferably the polypeptide or the functionally equivalent variant thereof, is used for the prevention and/or treatment of a human patient, it will be administered in quantities ranging from 0.1 mg/kg/day to 15 mg/kg/day, preferably 1 mg/kg/day to 14 mg/kg/day, more preferably 2 mg/kg/day to 13 mg/kg/day, more preferably 3 mg/kg/day to 12 mg/kg/day, more preferably 4 mg/kg/day to 11 mg/kg/day, more preferably 5 mg/kg/day to 10 mg/kg/day, more preferably 6 mg/kg/day to 9.5 mg/kg/day more preferably 6.08 mg/kg/day to 9 mg/kg/day. In a preferred embodiment, is administered three times per week or is administered 4 doses every other day, more preferably is intravenously administered.

In another particular embodiment, when the compound comprising the sequence SEQ ID NO: 1, the functionally equivalent variant thereof, the fusion protein, the conjugate, polynucleotide, vector or cell, preferably the polypeptide or the functionally equivalent variant thereof, is used for the prevention and/or treatment of a human patient, it will be administered in quantities ranging from 50 to 400 mg/m², preferably 100 to 380 mg/m², more preferably 110 to 370 mg/m², more preferably 120 to 360 mg/m², more preferably 130 to 350 mg/m², more preferably 140 to 340 mg/m², more preferably 150 to 330 mg/m², more preferably 160 to 320 mg/m², more preferably 170 to 310 mg/m², more preferably 180 to 300 mg/m², more preferably 190 to 290 mg/m², more preferably 200 to 275 mg/m², more preferably 220 to 250 mg/m², more preferably 210 to 230 mg/m². In a preferred embodiment, is administered three times per week or 4 doses every other day, more preferably is intravenously administered.

The compound for use or the pharmaceutical composition comprising the compound for use according to the invention can be administered by any type of suitable route, such as by oral route, topical route, by inhalation or parenteral route so that the pharmaceutically acceptable excipients necessary for the formulation of the desired dosage form will be included. Other routes of administration can be rectally, intracisternally or intravaginally.

In an embodiment, the compound for use according to the invention or the pharmaceutical composition comprising the compound for use is administered system ically.

"Systemic route" is understood as the administration by oral route, intravenous route, intraperitoneal route and intramuscular route. The amount of the compound required for the therapeutic or prophylactic effect will naturally vary according to the elected compound, the nature and the severity of the illness that is going to be treated, and the patient.

"Oral route" is understood as the compound or pharmaceutical composition incorporated into the organism after deglutition. In a particular embodiment, the compound or pharmaceutical composition of the invention can be in a dosage form suitable for its administration by oral route, whether it is solid or liquid. The dosage forms suitable for their administration by oral route can be tablets, capsules, syrups or solutions, and can contain any conventional excipient known in the art, such as binders, for example syrup, acacia, gelatin, sorbitol or polyvinylpyrrolidone; filling agents, for example lactose, sugar, corn starch, calcium phosphate, sorbitol or glycine; lubricants for compression, for example, magnesium stearate; disintegrating agents, for example starch, polyvinylpyrrolidone, sodium glycolate of starch or microcrystalline cellulose; or pharmaceutically acceptable wetting agents such as sodium lauryl sulfate. The solid oral compositions can be prepared by means of conventional processes of mixing, filling or compressing. Repetitive mixing operations can be used to completely distribute the active agent in those compositions that use high amounts of filling agents. Said operations are conventional in the art. The tablets can be prepared, for example, by means of wet or dry granulation, and optionally coating them according to the processes known in the common pharmaceutical practice, particularly with an enteric coating.

As it is used herein, the term "parenteral", includes administration by intravenous route, intraperitoneal route, intramuscular route or subcutaneous route. In a preferred embodiment, the compound for use according to the invention or the pharmaceutical composition comprising the compound is administered by intravenous, intraperitoneal, intramuscular or subcutaneous route, preferably by intraperitoneal route or intravenous route; more preferably by intravenous route. In an embodiment the administration is by subcutaneous infusion.

In one embodiment, the compound or pharmaceutical compositions comprising the compound for use according to the invention can be adapted for their parenteral administration, such as sterile solutions, suspensions or lyophilized products in the appropriate dosage unit form. The compound or pharmaceutical compositions suitable for its injectable use include sterile aqueous solutions (when they are soluble in water), or dispersions and sterile powders for the extemporaneous preparation of sterile injectable solutions or dispersions. For its administration by intravenous route, some suitable carriers include saline solution buffered with phosphate (PBS). In all the cases, the compound or composition must be sterile, and must be fluid to the point which that there exists easy ability for being injected. It must be stable in the preparation and storage conditions, and must be protected from the contamination action of microorganisms such as bacteria and fungi. The carrier can be a solvent or a dispersion medium which contains, for example, water, ethanol, a pharmaceutically acceptable polyol such as glycerol, propylene glycol, liquid polyethylene glycol and suitable mixtures thereof. Suitable fluidity can be maintained, for example, by means of using a coating such as lecithin, by means of maintaining the particle size required in the case of dispersion and by means of using surfactants. The prevention of the action of the microorganisms can be achieved by means of various antibacterial and antifungal agents, for example, parabens, chlorobutanol, phenol, ascorbic acid, thiomersal, and the like. In most cases, it will be preferable to include isotonic agents, for example, sugars; polyalcohols such as mannitol, sorbitol; or sodium chloride in the composition. The prolonged absorption of the injectable compositions may be caused by the inclusion of an agent which delays the absorption, for example, aluminium and gelatin monostearate.

The injectable sterile solutions can be prepared by incorporating the active compound in the required amount in a suitable solvent with one or a combination of the aforementioned ingredients, as needed, followed by sterilization by filtration through sterile membranes. Generally, the dispersions are prepared by incorporating the active compound in a sterile vehicle containing a basic dispersion medium and the rest of the ingredients required from among those previously listed. In the case of sterile powders for the preparation of injectable sterile solutions, the preferred preparation processes are vacuum drying and lyophilization which give rise to a powder with the active ingredient plus any desired additional ingredient from a previously filtered sterile solution thereof.

The compounds or pharmaceutical compositions of the invention can be suitably administered by means of pulse infusion, for example, with decreasing doses of the composition.

In a particular embodiment, the compound for use according to the invention or the pharmaceutical composition comprising the compound is administered intradermally, subcutaneously, intravenously, intraperitoneally, intramuscularly, intranasally, orally, or topically.

In a more particular embodiment, the compound for use according to the invention or the pharmaceutical composition comprising the compound is administered intravenously or intraperitoneally; preferably intravenously.

Intravenous delivery forms include, but are not limited to, bolus and drip injections. Examples of intravenous dosage forms include, but are not limited to, those contained in water, preferably sterile water for injection USP; aqueous vehicles including, but not limited to, sodium chloride injection, Ringer's injection, dextrose injection, dextrose and sodium chloride injection, and lactated Ringer's injection; water-miscible vehicles including, but not limited to, ethyl alcohol, polyethylene glycol and polypropylene glycol; and non-aqueous vehicles including, but not limited to, corn oil, cottonseed oil, peanut oil, sesame oil, ethyl oleate, isopropyl myristate and benzyl benzoate.

Intraperitoneal delivery forms include, but are not limited to, injections.

On the other hand, "topical route" is understood as an administration by non-systemic route, and includes the application of a compound or pharmaceutical composition of the invention externally on the epidermis, in the oral cavity and the instillation of said composition into ears, eyes and nose, and in which it does not significantly enter the blood stream. Dosage forms for topical or transdermal administration of a compound of this invention include ointments, pastes, creams, lotions, gels, powders, solutions, sprays, inhalants or patches.

Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

In another embodiment, the compound or pharmaceutical composition is administered intranasally. In a preferred embodiment, the intranasal administration is performed by instillation or nasal inhalation.

"Inhalation" is understood as the administration by intranasal route and by oral inhalation. The dosage forms suitable for said administration, such as a formulation in aerosol or a meter dosed inhaler can be prepared by means of conventional techniques. In an embodiment the route of administration is the intranasal route.

Dosage forms of compositions intended for intranasal and intrapulmonary administration are preferably a liquid, a suspension or a solid. A suspension is a liquid preparation containing solid particles dispersed in a liquid vehicle. The dosage forms are preferably metered. For example, metered drops/sprays mean that the dispenser that includes the drops/spray delivers the drops/spray containing a metered dose (a predetermined quantity) of the compound or composition for use according to the invention.

One preferred dosage form in the context of the intranasal administration route includes nasal drops. Drops are deposited mostly in the posterior portion of the nose and thus removed rapidly into the nasal pharynx. A concern with drops is often how to precisely control the drug's dose which is particularly important for the administration of the compound or composition.

Another intranasal dosage form by which the compound or the pharmaceutical composition for use according to the invention can be administered is nasal sprays. Nasal sprays typically contain the compound dissolved or suspended in a solution or a mixture of excipients (e.g. preservatives, viscosity modifiers, emulsifiers, buffering agents) in a non-pressurized dispenser. Nasal sprays have several advantages including compactness of the delivery device, convenience, simplicity of use, and accuracy of delivering dosages of 25 to 200 pL. They are deposited in the anterior portion of the nose and cleared slowly into nasal pharynx by mucociliary clearance. The nasal spray as used herein can be a liquid or a suspension.

Another intranasal dosage form is a nasal aerosol. Nasal aerosols differ from nasal sprays by the method of the composition dispensing: in aerosols, a compound is dispensed due to an excess of pressure and releases through a valve. In sprays, a compound is dispensed due to forcing away by a micropump bucket, while the pressure in the vial is similar to atmosphere pressure. Aerosols have similar advantages as sprays.

The compound or composition for use according to the invention may alternatively preferably be administered by nasal emulsions, ointments, gels, pastes or creams. These are highly viscous solutions or suspensions applied to the nasal mucosa.

Due to the limited volume of the composition that can be efficiently delivered to the nasal mucosa, liquid intranasal dosage forms usually have higher concentrations as the corresponding intravenous dosage forms. When substances become poorly soluble or are instable in liquid form, powders can be used to administer the compound or the composition for use of the invention. Further advantages of powders are that they do not require preservatives and have usually a higher stability as compared to liquid formulations. The main limitation on intranasal powder application is related to its irritating effect on the nasal mucosa.

One dosage form in context of intrapulmonary administration is an inhalation aerosol. Inhalation aerosols are usually packaged under pressure and contain the compound or composition for use according to the invention which is released upon activation of a valve system into the respiratory tract, in particular the lungs. The released aerosol is a colloid of fine solid particles (suspension) or liquid droplets (solution) in air or another gas. Accordingly, the aerosol may be a solution or a suspension aerosol. The liquid droplets or solid particles have preferably a diameter of less than 100 pm, more preferably less than 10 pm, most preferably less than 1 pm.

Another dosage form in context of intrapulmonary administration is inhalation sprays. Inhalation sprays are typically aqueous based and do not contain any propellant. They deliver the conjugate to the lungs by oral inhalation.

Nebulized inhalation solutions and suspensions may also be used to deliver the compound or composition by the intrapulmonary route. Nebulized inhalation solutions and suspensions are typically aqueous-based formulations that contain the compound or composition for use according to the invention. The nebulized inhalation solutions and suspensions deliver the compound or composition to the lungs by oral inhalation for systemic effects and are used with a nebulizer.

Dry powder inhalation is an alternative to aerosol inhalation. The composition is usually included in a capsule for manual loading or within the inhaler. Dry powders are typically delivered by an inhaler to the lungs by oral inhalation. The dry powders as used herein can be formulated neat. Neat formulations contain the drug alone or quasi-alone e.g. as spry dried powder. The dry powders as used herein can be also formulated with a carrier such as lactose.

Intrapulmonary dosage forms are preferably metered, i.e. are delivered to the lungs in a predetermined quantity.

Devices for intranasal delivery in the context of the present invention include spray pump systems, pipettes for delivering drops, metered-dose spray pumps, nasal pressurized metered-dose inhalers, powder spray systems, breath-actuated powder inhalers and nasal powder insufflators. The intranasal delivery device may be filled with a single dose amount or a multi-dose amount of the intranasal formulation.

Using the intrapulmonary route the compound or composition may be administered with a metered dose inhaler. A metered-dose inhaler (MDI) provides a fine mist of compound, generally with an aerodynamic particle size of less than 5 pm.

Dry powder inhalers can be alternatively used to deliver the compound or composition intrapulmonary. Dry powder inhalers present powders as single-dose or multidose powders.

Another device for intrapulmonary delivery is a nebulizer including ultrasonic and air jet nebulizers. In ultrasonic nebulizers, ultrasound waves are formed in an ultrasonic nebulizer chamber by a ceramic piezoelectric crystal that vibrates when electrically excited. This generates an aerosol cloud at the solution surface. The aerosol produced by an air jet nebulizer is generated when compressed air is forced through an orifice. A liquid may be withdrawn from a perpendicular nozzle (the Bernoulli Effect) to mix with the air jet which is atomized using baffles to facilitate the formation of the aerosol cloud.

In one embodiment, the compound or the pharmaceutical composition for use according to the invention is prepared with carriers which will protect the components from a rapid elimination from the body, such as a controlled release formulation, including implants and microencapsulated administration systems. Biodegradable biocompatible polymers such as ethylene vinylacetate, polyanhydrides, polyglycolic acid, collagen, polyorthoesters and polylactic acid can be used. The processes for preparing said formulations will be clear for persons skilled in the art. The materials can also be commercially obtained in Alza Corporation and Nova Pharmaceuticals, Inc.

The sustained release compositions also include preparations of crystals suspended in suitable formulations which can maintain the crystals in suspension. These preparations, when they are injected by subcutaneous or intraperitoneal route may produce a sustained release effect. Other compositions also include the components trapped in liposomes. The liposomes containing such components are prepared by means of known methods such as Epstein et al., Proc. Natl. Acad. Sci. USA, (1985) 82:3688-3692; Hwang et al., Proc. Natl. Acad. Sci. USA, (1980) 77:4030-4034; EP 52,322; EP 36,676; EP 88,046; EP 143,949. In a preferred embodiment, the compound of the invention is contained in liposomes.

Despite the fact that Omomyc, functionally equivalent variants thereof, conjugates and fusion proteins of the invention are capable of translocating across biological membranes, it is possible to formulate Omomyc, any of its functionally equivalent variants, conjugates, polynucleotides, vectors or cells in nanoparticles. The nanoparticles may contribute to preserve the integrity of the components in the biological fluids until it reaches the target organ. In addition, nanoparticles can also be modified so as to include moieties which allow the targeting of the nanoparticle to an organ of interest. In this way, the compound of the invention will be delivered in the proximity of the target organ, facilitating access of the compound to the interior of the cells where its biological activity is required.

Thus, in another embodiment, the compound of the invention is provided forming part of a nanoparticle.

As used herein, the term "nanoparticle" refers to any material having dimensions in the 1-1,000 nm range. In some embodiments, nanoparticles have dimensions in the 2-200 nm range, preferably in the 2-150 nm range, and even more preferably in the 2-100 nm range. Nanoparticles that can be used in the present invention include such nanoscale materials as a lipid-based nanoparticle, a superparamagnetic nanoparticle, a nanoshell, a semiconductor nanocrystal, a quantum dot, a polymer-based nanoparticle, a silicon-based nanoparticle, a silica-based nanoparticle, a metal-based nanoparticle, a fullerene and a nanotube. Molecules can be either embedded in the nanoparticle matrix or may be adsorbed onto its surface, preferably molecules are embedded in the nanoparticle.

In a preferred embodiment, the nanoparticle is a liposome.

Targeted delivery can be achieved by the addition of ligands without compromising the ability of nanoparticles to deliver their content. It is contemplated that this will enable delivery to specific cells, tissues and organs. The targeting specificity of the ligand-based delivery systems are based on the distribution of the ligand receptors on different cell types. The targeting ligand may either be non-covalently or covalently associated with a nanoparticle, and can be conjugated to the nanoparticles by a variety of methods as discussed herein.

Examples of proteins or peptides that can be used to target nanoparticles include transferin, lactoferrin, TGF-β, nerve growth factor, albumin, HIV Tat peptide, RGD peptide, and insulin, as well as others.

It will be understood that the formulations of the invention in a nanoparticle are not intended or are not solely intended for facilitating the access of the components to the interior of the cell but to protect components from degradation and/or for facilitating targeting of the nanoparticle to the organ of interest.

In one example, the nanoparticle may be made up of a biodegradable polymer such as poly(butylcyanoacrylate) (PBCA). Examples of elemental nanoparticles include carbon nanoparticles and iron oxide nanoparticles, which can then be coated with oleic acid (OA)-Pluronic(R). In this approach, a drug (e.g., a hydrophobic or water insoluble drug) is loaded into the nanoparticle. Other nanoparticles are made of silica.

Nanoparticles can be formed from any useful polymer. Examples of polymers include biodegradable polymers, such as poly(butyl cyanoacrylate), poly(lactide), poly(glycolide), poly-s-caprolactone, poly(butylene succinate), poly(ethylene succinate), and poly(p-dioxanone); poly(ethyleneglycol); poly-2-hydroxyethylmethacrylate (poly(HEMA)); copolymers, such as poly(lactide-co-glycolide), poly(lactide)-poly(ethyleneglycol), poly(poly(ethyleneglycol)cyanoacrylate-cohexadecylcyanoacrylate, and poly [HEMA-co-methacrylic acid]; proteins, such as fibrinogen, collagen, gelatin, and elastin; and polysaccharides, such as amylopectin, a amylose, and chitosan.

Other nanoparticles include solid lipid nanoparticles (SLN). Examples of lipid molecules for solid lipid nanoparticles include stearic acid and modified stearic acid, such as stearic acid-PEG 2000; soybean lecithin; and emulsifying wax. Solid lipid nanoparticles can optionally include other components, including surfactants, such as Epicuron(R) 200, poloxamer 188 (Pluronic(R) F68), Brij 72, Brij 78, polysorbate 80 (Tween 80); and salts, such as taurocholate sodium. Agents can be introduced into solid lipid nanoparticles by a number of methods discussed for liposomes, where such methods can further include high-pressure homogenization, and dispersion of microemulsions.

Nanoparticles can also include nanometer-sized micelles. Micelles can be formed from any polymers described herein. Exemplary polymers for forming micelles include block copolymers, such as poly(ethylene glycol) and poly($\epsilon$-caprolactone). (e.g.,a PEO- b-PCL block copolymer including a polymer of $\epsilon$-caprolactone and $\alpha$-methoxy-$\omega$-hydroxy-poly(ethylene glycol)).

In certain embodiments, the properties of the nanoparticles are altered by coating with a surfactant. Any biocompatible surfactant may be used, for example, polysorbate surfactants, such as polysorbate 20, 40, 60, and 80 (Tween 80); Epicuron(R) 200; poloxamer surfactants, such as 188 (Pluronic(R) F68) poloxamer 908 and 1508; and Brij surfactants, such as Brij 72 and Brij 78.

Nanoparticles can optionally be modified to include hydrophilic polymer groups (e.g., poly(ethyleneglycol) or poly(propyleneglycol)), for example, by covalently attaching hydrophilic polymer groups to the surface or by using polymers that contain such hydrophilic polymer groups (e.g., poly[methoxy poly (ethyleneglycol) cyanoacrylate-co-hexadecyl cyanoacrylate]). Nanoparticles can be optionally cross linked, which can be particularly useful for protein-based nanoparticles.

In another embodiment, the pharmaceutical composition of the invention is a nanoemulsion. "Nanoemulsion" as used herein means a colloidal dispersion of droplets (or particles) which at least some of the droplets have diameters in the nanometer size range. The nanoemulsions are comprised of omega-3, -6 or -9 fatty acid rich oils in an aqueous phase and thermo-dynamically stabilized by amphiphilic surfactants, which make up the interfacial surface membrane, produced using a high shear microfluidization process usually with droplet diameter within the range of about 80-220 nm.

In a particular embodiment, the compound for use according to the invention is administered in combination with at least another therapeutic agent useful in the treatment of an autoimmune disease, wherein said at least another therapeutic agent is administered simultaneously, sequentially or separately.

"Simultaneous administration" encompasses coadministration of the two therapeutic agents, regardless of the relative frequencies or timing of the administration of the respective agents. Thus, simultaneous administration encompasses the coadministration of the two therapeutic agents at the same time and at the same frequencies of administration. In addition, simultaneous administration refers to the coadministration of the two therapeutic agents, in which one agent is administered more frequently than the other(s). In addition, simultaneous administration refers to the coadministration of the two therapeutic agents, in which one agent is administered only once during the administration of the other agent(s).

"Sequentially" administration occurs when the administration of the first component is discontinued before starting with the administration of the second component.

If administered separately, the therapeutic agents can be administered within a period of time from one another, for example, within 1, 2, 3, 4, 5, 6, 7, 8, 9, 10, 11, 12, 13, 14, 15, 16, 17, 18, 19, 20, 21, 22, 23, or 24 hours from one another. In some embodiments, can be administered within 1, 2, 3, 4, 5, 6, 7, 8, 9, 10, 11, 12, 13, 14, 15, 16, 17, 18, 19, 20, 21, 22, 23, or 24 days from one another.

Combinations and pharmaceutical compositions of the invention

In another aspect, the invention relates to a combination comprising:

(i) a compound according to the invention and

(ii) an agent useful in the treatment of an autoimmune disease.

In another aspect, the invention relates to a pharmaceutical composition comprising a pharmaceutically effective amount of a combination according to the invention and a pharmaceutically acceptable excipient.

In another aspect, the invention relates to a combination according to the invention or a pharmaceutical composition according to the invention for use in medicine.

In another aspect, the invention relates to a combination according to the invention or a pharmaceutical composition according to the invention for use in the prevention and/or treatment of an autoimmune disease.

The embodiments defined in relation to the first aspect of the invention are also applicable to these additional aspects.

Furthermore, the embodiments defined in the context of the combinations and pharmaceutical compositions of the invention are also applicable to the first aspect of the invention.

According to the invention, the expression "combination" stands for the various combinations of compounds (i) and (ii), for example in a composition formulated as a single formulation, in a combined mixture composed from separate formulations of each of the components, such as a "tank-mix" which may be combined for joint use as a combined preparation, and in a combined use of the single active ingredients when applied in a sequential manner, i.e., one after the other with a reasonably short period, such as a few hours or days or in simultaneous administration. In the present invention, compound (i) refers to a therapeutically effective amount of a polypeptide comprising the sequence SEQ ID NO: 1 or a functionally equivalent variant thereof, or refers to a conjugate comprising a polypeptide comprising the sequence SEQ ID NO: 1 or a functionally equivalent variant thereof and a chemical moiety that facilitates cellular uptake of the polypeptide or of the functionally equivalent variant thereof, or refers to a polynucleotide encoding the polypeptide or the conjugate, or refers to a vector comprising the polynucleotide, or refers to a cell capable of secreting into the medium the polypeptide or the conjugate. In the present invention, compound (ii) refers to a therapeutically effective amount of a compound suitable for the treatment of an autoimmune disease. Preferably, the order of applying the compounds (i) and (ii) is not essential for working the present invention.

The combination may be a kit-of-parts wherein each of the components is individually formulated and packaged.

The combination of (i) and (ii) can be formulated for its simultaneous, separate or sequential administration as explained previously.

Particularly, if the administration is not simultaneous, the compounds are administered in a close time proximity to each other. Furthermore, compounds are administered in the same or different dosage form or by the same or different administration route, e.g. one compound can be administered orally and the other compound can be administered intravenously. Preferably, compound (i) is administered intravenously and compound (ii) is administered systemically, more preferably parenterally, even more preferably orally.

The combination of the two compounds (i) and (ii) can be administered:

- as a combination that is being part of the same medicament formulation, the two compounds being then administered always simultaneously.

- as a combination of two units, each with one of the substances giving rise to the possibility of simultaneous, sequential or separate administration.

In a particular embodiment, compound (i) of the combination of the invention is independently administered from compound (ii), i.e. in two units, but at the same time.

In another particular embodiment, compound (i) of the combination of the invention is administered first, and then compound (ii), i.e. the compound (ii) is separately or sequentially administered.

The pharmaceutical compositions according to the invention, which contain a compound according to the invention and a compound suitable for the treatment of an autoimmune disease may be presented as a single formulation (for example, as a tablet or a capsule comprising a fixed quantity of each one of the components) or can, on the other hand, be presented as separate formulations to be later combined for joint, sequential, or separate administration. The compositions of the invention also include the formulation as a kit-of-parts wherein the components are formulated separately but are packaged in the same container. Those skilled in the art will appreciate that the formulation of the different components in the pharmaceutical composition according to the invention may be similar, in other words, similarly formulated (in tablets or pills), which allows their administration by the same route. In the case where the different components of the invention are formulated separately, the two components can be presented in a blister. Each blister contains the drugs that must be consumed during the day. If the drugs must be administered several times a day, the drugs corresponding to each administration can be placed in different sections of the blister, preferably recording in each section of the blister the time of day when they should be administered. Alternatively, the components of the composition of the invention can be formulated differently so that the different components are differently administered. Thus, it is possible that the first component is formulated as a tablet or capsule for its oral administration and the second component is formulated for its intravenous administration or vice versa. The ratio between the components that are part of the combinations or pharmaceutical compositions according to the invention can be adjusted by the skilled person depending on the agent used in each particular case, as well as of the desired indication. Thus, the invention envisages compositions wherein the ratio between the quantities of component (i) and component (ii) can range from 50:1 to 1:50, in particular from 20:1 to 1:20, from 1:10 to 10:1, or from 5:1 to 1:5. In a more particular embodiment, the ratio between quantities ranges from 1:1 to 1:5, preferably from 1:1 to 1:3. In a more preferred embodiment, the ratio ranges from 1:1 to 1:1.5, preferably from 1:1.3 to 1:1.4, more preferably 1:1.34. In another preferred embodiment, the ratio ranges from 1:1 to 1:2.8, preferably from 1:2.6 to 1:2.7, more preferably 1:2.67. In another particular embodiment, the ratio between quantities ranges from 30:1 to 5:1, preferably from 30:1 to 8:1, more preferably from 25:1 to 15:1, more preferably from 20:1 to 10:1. In an embodiment, the ratio is 20:1. In another embodiment, the ratio is 10:1. Preferably these ratios are w/w ratios.

The expression "therapeutic agent useful in the treatment of an autoimmune disease", as used herein, refers to an agent suitable to be used to treat one of the diseases mentioned above.

In a more particular embodiment, the at least another agent useful in the treatment of an autoimmune disease is selected from the group consisting of a nonsteroidal anti-inflammatory drug (NSAID), a steroid and an immunosuppressant.

In a more particular embodiment, the at least another therapeutic agent useful in the treatment of an autoimmune disease is selected from the group consisting of: corticosteroids, ofatumumab, fingolimod, siponimod, ozanimod, ponesimod, laquinimod, teriflunomide, fumaric acid esters (dimethyl fumarate, diroximel fumarate, monomethyl fumarate), cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig, and atacicept. In a preferred embodiment, the at least another therapeutic agent for treating multiple sclerosis is selected from the group consisting of: corticosteroids, ofatumumab, fingolimod, siponimod, ozanimod, ponesimod, laquinimod, teriflunomide, fumaric acid esters (dimethyl fumarate, diroximel fumarate, monomethyl fumarate), cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig, copaxone, tocilizumab and atacicept. In a more preferred embodiment the at least another therapeutic agent for treating multiple sclerosis is selected from the group consisting of interferon, copaxone, teriflunomide, fumarate, natalizumab, fingolimod, alemtuzumab, rituximab, ocrelizumab, cladribine, ponesimod, siponimod, ozanimod, tocilizumab and ofatumumab.

In a preferred embodiment, the therapeutic agent for treating systemic lupus erythematosus, preferably lupus nephritis, is selected from the group consisting of cyclophosphamide, mycophenolate mofetil, calcineurin inhibitors, rituximab, ocrelizumab, belimumab, atacicept, abatacept, alemtuzumab, sirukumab, tocilizumab, etanercept, eculizumab, epratuzumab, abetimus/LJP-394, BG9588/IDEC 131, intravenous immunoglobulins, hydroxychloroquine, tacrolimus and corticoids.

In another preferred embodiment, the therapeutic agent for treating rheumatoid arthritis is selected from the group consisting of infliximab, adalimumab, certolizumab, golimumab and etanercept.

In another preferred embodiment, the therapeutic agent for treating inflammatory bowel disease is selected from the group consisting of cyclosporine A, tacrolimus, methotrexate, thiopurines and anti-TNF agents.

Therapeutic agents for the treatment of autoimmune diseases, particularly diabetes mellitus (type I), can be selected from the group consisting of insulin, teplizumab, otelixizumab, alefacept, abatacept, rituximab, infliximab, adalimumab, etanercept, golimumab, tocilizumab, verapamil, dapagliflozin, empagliflozin, sotagliflozin, exenatide, liraglutide, dulaglutide and semaglutide.

Therapeutic agents for the treatment of autoimmune diseases, particularly Grave's disease, can be selected from the group consisting of anti-thyroid medications, such as propylthiouracil and methimazole, beta blockers such as propranolol, atenolol, metoprolol and nadolol, corticosteroids, and teprotumumab.

Therapeutic agents for the treatment of autoimmune diseases, particularly alopecia areata, can be selected from the group consisting of topical corticosteroids, intralesional corticosteroids, anthralin, minoxidil, topical tacrolimus, topical retinoids, prostaglandin analogues, azathioprine, cyclosporine, methotrexate and sulfasalazine.

Therapeutic agents for the treatment of autoimmune diseases, particularly Addison's disease, can be selected from the group consisting of hydrocortisone, prednisone, methylprednisolone and fludrocortisone acetate.

Therapeutic agents for the treatment of autoimmune diseases, particularly psoriasis, can be selected from the group consisting of corticosteroids, vitamin D analogues (such as calcipotriene and calcitriol), retinoids (such as tazarotene), calcineurin inhibitors (such as tacrolimus and pimecrolimus), salicylic acid, anthralin, steroids, retinoids, apremilast (Otezla®), etanercept (Enbrel®), infliximab (Remicade®), adalimumab (Humira®), ustekinumab (Stelara®), secukinumab (Cosentyx®), ixekizumab (Taltz®), guselkumab (Tremfya®), tildrakizumab (Ilumya®) and certolizumab (Cimzia®), methotrexate, cyclosporine, thioguanine (Tabloid®) and hydroxyurea (Droxia®, Hydrea®).

Therapeutic agents for the treatment of autoimmune diseases, particularly autoimmune renal diseases, more particularly lupus nephritis can be selected from the group consisting of immunosuppressors, steroids, vitamin D, VIP (vasoactive intestinal peptide), hydroxychloroquine, chloroquine and T cell vaccination.

In an embodiment, the immunosuppressors are selected from the group consisting of cyclophosphamide, mycophenolate mofetil, azathioprine and anticalcineurinics.

In an embodiment, the steroids are selected from the group consisting of prednisolone and methylprednisolone.

Therapeutic agents for the treatment of autoimmune diseases, particularly autoimmune renal disease, more particularly lupus nephritis can be compounds targeting a target selected from the group consisting of T cells, B cells, co-stimulation inhibition, inflammatory cytokines, cell adhesion molecules and complement components.

In an embodiment, the T cell target can be selected from the group consisting of CD3, CD52, IL-2 and LFA-1. Preferably, the compounds that target T cells are selected from the group consisting of OKT3, alemtuzumab, basiliximab, efalizumab, laquinimod and rapamycin.

In another embodiment, the B cell target can be selected from the group consisting of CD20, CD22, BAFF, BAFF and APRIL, FcγRIlb and CD74. Preferably, the compounds that target B cells are selected from the group consisting of rituximab, ocrelizumab, oftumumab, veltuzumab, obinutuzumab, epratuzumab, belimumab, blisibimod, atacicept, soluble FcyRIlb, milatuzumab and tabalumab.

In an embodiment the co-stimulation inhibition can be selected from the group consisting of CD40 and CD80/86. Preferably, the compounds that target co-stimulation inhibition are selected from the group consisting of ASKP1240, abatacept and, belatacept.

In an embodiment, the inflammatory cytokines target can be selected from the group consisting of IL-1β, IL-6, IL-6R, TNF, TWEAK, IFNα, IL-17A, IL-12/IL-23, IFNγ, MIF and IL-5. Preferably, the compounds that target inflammatory cytokines are selected from the group consisting of canakinumab, anakinra, sirukumab, tocilizumab, etanercept, infliximab, adalimumab, golimumab, certolizumab, BIIB023, sifalimumab, rontalizumab, secukinumab, ustekinumab, AMG 811, imalumab, mepolizumab, brodalumab, briakinumab, sarilumab, rilonacept and anifrolumab.

In an embodiment, the cell adhesion molecules target is VLA-1. Preferably, the compound that targets cell adhesion molecules is natalizumab.

In an embodiment, the complement component target is selected from the group consisting of C5 and C5aR. Preferably, the compounds that target complement components are selected from the group consisting of eculizumab, mubodina, LFG316 and CCX168.

Table 2 shows the different compounds mentioned above and their target.

**Table 2. Compounds for the treatment of autoimmune diseases. Adapted from** Holdsworth S.R. et al. 2016. Nat. Rev. 12:217-231.

| Target | | Compound |
|---|---|---|
| _T cells_ | CD3 | OKT3 |
| | CD52 | alemtuzumab |
| | IL-2 | Basilximab rapamycin |
| | LFA-1 | efalizumab |
| _B cells_ | CD20 | rituximab ocrelizumab oftumumab veltuzumab obinutuzumab |
| | CD22 | epratuzumab |
| | BAFF | belimumab blisibimod tabalumab |
| | BAFF and APRIL | atacicept |
| | FCγRIlb | soluble FCγRIlb |
| | CD74 | milatuzumab |

(continued)

| Target | | Compound |
|---|---|---|
| *Co-stimulation inhibition* | CD40 | ASKP1240 |
| | CD80/86 | abatacept<br>belatacept |
| *Inflammatory cytokines* | IL-1β | canakinumab<br>anakinra<br>rilonacept |
| | IL-6 | sirukumab |
| | IL-6R | tocilizumab<br>sarilumab |
| | TNF | etanercept<br>infliximab<br>adalimumab<br>golimumab<br>certolizumab |
| | TWEAK | BIIB023 |
| | IFNα | sifalimumab<br>rontalizumab<br>anifrolumab |
| | IL-17A | secukinumab<br>brodalumab |
| | IL-12/IL-23 | ustekinumab<br>briakinumab |
| | IFNγ | AMG 811 |
| | MIF | imalumab |
| | IL-5 | mepolizumab |
| *Cell adhesion molecules* | VLA-1 | natalizumab |
| *Complement components* | C5 | eculizumab<br>mubodina<br>LFG316 |
| | C5aR | CCX168 |

In a preferred embodiment, the therapeutic agent useful in the treatment of an autoimmune disease is selected from the group consisting of cyclosporine A, tacrolimus, methotrexate, thiopurines, anti-TNF agents, infliximab, adalimumab, certolizumab, golimumab, etanercept, rituximab, epratuzumab, belimumab, rapamycin, anti-interferon antibodies, tocilizumab, laquinimod, tabalumab, ofatumumab, ixekizumab, brodalumab, briakinumab, sarilumab, rilonacept, anifrolumab, cyclophosphamide, mycophenolate mofetil, azathioprine, anticalcineurinics, prednisolone, methylprednisolone, vitamin D, vasoactive intestinal peptide, hydroxychloroquine, chloroquine, ocrelizumab, atacicept, abatacept, alemtuzumab, sirukumab, eculizumab and T cell vaccine.

In further aspects, the present invention relates to:

1. A compound selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,
(c) a polynucleotide encoding the polypeptide of a) or the conjugate of b),

(d) a vector comprising the polynucleotide according to c), and

(e) a cell capable of secreting into the medium the polypeptide according to a) or the conjugate according to b)

for use in the prevention and/or treatment of an autoimmune disease in a subject.

2. The compound for use according to aspect 1, wherein the functionally equivalent variant of SEQ ID NO: 1 is selected from the group consisting of SEQ ID NO: 4, SEQ ID NO: 5, SEQ ID NO; 6, SEQ ID NO: 7, SEQ ID NO: 8, SEQ ID NO: 9, and SEQ ID NO: 10.

3. The compound for use according to any one of aspects 1 or 2, wherein the chemical moiety that facilitates the cellular uptake of the polypeptide or the functionally equivalent variant thereof is a cell-penetrating peptide sequence and wherein said cell penetrating peptide sequence and said polypeptide or functionally equivalent variant thereof form a fusion protein.

4. The compound for use according to aspect 3, wherein the cell-penetrating peptide sequence is selected from the group consisting of SEQ ID NO: 15, SEQ ID NO: 16, SEQ ID NO: 20, SEQ ID NO: 21, SEQ ID NO: 22, SEQ ID NO: 37 and SEQ ID NO: 38.

5. The compound for use according to any one of aspects 1 to 4, wherein the conjugate additionally comprises a further nuclear localization signal.

6. The compound for use according to any one of aspects 1 to 5, wherein the compound is a polypeptide comprising the sequence SEQ ID NO: 1.

7. The compound for use according to any one of aspects 1 to 6, wherein the subject is a mammal.

8. The compound for use according to aspect 7, wherein the subject is a human.

9. The compound for use according to any one of aspects 1 to 8, wherein the autoimmune disease is selected from the group consisting of: celiac disease, diabetes mellitus (type I), Latent Autoimmune Diabetes in Adults (LADA), Graves' disease, inflammatory bowel disease, multiple sclerosis, alopecia areata, Addison's disease, pernicious anemia, psoriasis, rheumatoid arthritis, and systemic lupus erythematosus.

10. The compound for use according to aspect 9, wherein the autoimmune disease is selected from the group consisting of diabetes mellitus (type I), multiple sclerosis and psoriasis.

11. The compound for use according to aspect 10, wherein the autoimmune disease is multiple sclerosis.

12. The compound for use according to any one of aspects 1 to 8, wherein the autoimmune disease is a central nervous system (CNS) autoimmune demyelinating disease.

13. The compound for use according to aspect 12, wherein the CNS autoimmune demyelinating disease is selected from the group consisting of multiple sclerosis (MS), clinically isolated syndrome (CIS), tumefactive (tumor-like) MS, Marburg's acute MS, Balós's concentric sclerosis, acute disseminated encephalomyelitis (ADEM), acute hemorrhagic leukoencephalitis, Schilder's disease, solitary sclerosis, transverse myelitis, Susac's syndrome, leukoaraiosis, myalgic encephalomyelitis, Guillain-Barré syndrome, progressive inflammatory neurophathy, leukodystrophy, including adrenoleukodystrophy, adrenomyeloneuropathy, post-vaccinal encephalitis (PVE), post-infectious encephalomyelitis (PIE), neuromyelitis optica (NMO), optic neuritis and myelin oligodendrocyte glycoprotein (MOG)-associated disease (MOGAD).

14. The compound for use according to any one of aspects 1 to 13, wherein the compound is administered intradermally, subcutaneously,, intravenously, intraperitoneally, intramuscularly, intranasally, orally or topically.

15. The compound for use according aspect 14, wherein the compound is administered intravenously or intraperitoneally.

16. The compound for use according to any one of aspects 1 to 15, wherein the compound is administered at a dosage level between 0.1 and 100 mg/kg per day, preferably between 1 and 85 mg/kg per day, more preferably between 5 and 75 mg/kg per day.

17. The compound for use according to any one of aspects 1 to 16, wherein the compound is administered in combination with at least another therapeutic agent useful in the treatment of an autoimmune disease, wherein said at least another therapeutic agent is administered simultaneously, sequentially or separately.

18. The compound for use according to aspect 17, wherein the at least another therapeutic agent useful in the treatment of an autoimmune disease is selected from the group consisting of: corticosteroids, ofatumumab, fingolimod, siponimod, ozanimod, ponesimod, laquinimod, teriflunomide, fumaric acid esters (dimethyl fumarate, diroximel fumarate, monomethyl fumarate), cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig, copaxone, tocilizumab and atacicept.

19. The compound for use according to aspect 17, wherein the at least another therapeutic agent useful in the treatment of an autoimmune disease is selected from the group consisting of interferon, copaxone, teriflunomide, fumarate, natalizumab, fingolimod, alemtuzumab, rituximab, ocrelizumab, cladribine, ponesimod, siponimod, ozanimod, tocilizumab and ofatumumab.

20. The compound for use according to any one of aspects 1 to 19, wherein the compound is comprised within a pharmaceutical composition wherein the pharmaceutical composition further comprises at least one pharmaceutically acceptable excipient.

21. A combination comprising:

(i) a compound selected from the group consisting of:

(a) a polypeptide comprising the sequence SEQ ID NO: 1 or a functionally equivalent variant thereof,
(b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,
(c) a polynucleotide encoding the polypeptide of a) or the conjugate of b),
(d) a vector comprising the polynucleotide according to c), and
(e) a cell capable of secreting into the medium the polypeptide according to a) or the conjugate according to b); and

(ii) an agent useful in the treatment of an autoimmune disease.

22. The combination according to claim 21, wherein the agent useful in the treatment of an autoimmune disease is selected from the group consisting of interferon, copaxone, teriflunomide, fumarate, natalizumab, fingolimod, alemtuzumab, rituximab, ocrelizumab, cladribine, ponesimod, siponimod, ozanimod, tocilizumab and ofatumumab; more preferably selected from the group consisting of copaxone, teriflunomide, fumarate, natalizumab, fingolimod, ocrelizumab, ponesimod, siponimod, ozanimod, and tocilizumab.

23. A pharmaceutical composition comprising a pharmaceutically effective amount of a combination according to aspects 21 or 22 and a pharmaceutically acceptable excipient.

24. A combination according to aspects 21 or 22 or a pharmaceutical composition according to aspect 23 for use in medicine.

25. A combination according to aspects 21 or 22 or a pharmaceutical composition according to aspect 23 for use in the prevention and/or treatment of an autoimmune disease.

The invention is described herein by way of the following examples which are merely illustrative and not limitative of the scope of the invention.

**EXAMPLES**

**Material and methods**

Experimental autoimmune encephalomyelitis (EAE) induction and follow-up

Eight-week-old C57BL6/JOIaHsd females were purchased from Envigo (Netherlands). Anesthetised mice were immunised by subcutaneous injections of 100 $\mu$l phosphate buffered saline (PBS) containing 100$\mu$g of peptide 35-55 from myelin oligodendrocyte glycoprotein (MOG$_{35-55}$) emulsified in 100$\mu$l of complete Freund's adjuvant (incomplete Freund's adjuvant containing 4 mg/ml *Mycobacterium tuberculosis* H37RA). At 0- and 2-days post-immunization, mice were intravenously injected with 250ng of pertussis toxin. Mice were weighed and examined daily for neurological signs in a blinded manner using the following criteria: 0, no clinical signs; 0.5, partial paresis of tail; 1, paralysis of whole tail; 2, mild paraparesis of one or both hind limbs; 2.5, severe paraparesis or paraplegia of hind limbs; 3, mild tetraparesis (mild to moderate in hind limbs); 4, tetraparesis (severe in hind limbs); 4.5, severe tetraparesis; 5, tetraplegia; and 6, death (Gutierrez-Franco A., et al. 2017. Semaphorin 7A as a Potential Therapeutic Target for Multiple Sclerosis. Mol Neurobiol 54(6): 4820-4831). If weight loss was greater than 15%, mice received subcutaneously 0.5 ml of 10% glucose and score 5 and weight loss greater than 30% were defined as endpoint criteria to minimize the suffering and guarantee the animal welfare. All data presented are in accordance with the guidelines suggested for EAE publication (Baker D. and Amor S. 2012. Publication guidelines for refereeing and reporting on animal use in experimental autoimmune encephalomyelitis. J Neuroimmunol 242(1-2): 78-83). In the *in vitro* experiments, mice were followed for 20 days and then euthanized to obtain spleens. In the *in vivo* experiments, mice were followed for 28 days.

EAE clinical parameters

In the *in vivo* experiments, the area under the curve (AUC) of the mean EAE clinical score was calculated as a measure of disease severity. The loss of weight is a characteristic feature of EAE and deeply correlates with disease severity, so the AUC of the mean weight loss was also calculated and compared between groups. Other clinical parameters such as the maximum score reached along the clinical follow-up and the onset day were compared.

Statistical analysis

Statistical analysis was performed with GraphPad Prism 8 or SPSS Statistics v26 software packages. The *ex vivo* experiments were analyzed with a One-way ANOVA using Dunnett's as the post-hoc test. For the *in vivo* experiments, clinical parameters from vehicle- and Omomyc-treated groups were compared using a T-test.

Omomyc is able to reduce splenocyte proliferation and induce cell death in a dose dependent manner, both in PHA- and MOG-stimulated splenocytes from EAE-diseased mice (Figure 1). Spleens from EAE-diseased mice were collected 20 days post-immunization (when mice present clinical symptoms). Splenocytes were isolated and seeded in 96-well plates. Splenocyte cultures were stimulated with 5$\mu$g/ml MOG$_{35-55}$ (antigen-specific stimulation) or 5$\mu$g/ml PHA-L (polyclonal stimulation) in the presence of increasing concentrations of Omomyc (SEQ ID NO: 4) (1, 10, 100 and 200$\mu$g/ml). For the proliferation assay, five replicates per experimental condition (MOG$_{35-55}$, PHA-L and control) and sample were assessed. Cultures were maintained for 72h, adding 1 $\mu$Ci of $^3$H-thymidine to each well the last 18h. Incorporated radioactivity was measured in a beta-scintillation counter. Stimulation index (SI) was calculated by dividing the mean counts per minute (cpm) of MOG$_{35-55}$ or PHA-L conditions by the mean cpm of the control condition. After 48h of stimulation (5$\mu$g/ml MOG$_{35-55}$ or 5$\mu$g/ml PHA-L) in the presence of 1, 10, 100 and 200$\mu$g/ml Omomyc, cell death was assessed using a Fixable Viability Dye, and the percentage of death cells was determined by flow cytometry.

Omomyc treatment was able to delay disease onset and ameliorated the clinical course of the disease (Figure 2). To determine the potential therapeutic effect of Omomyc in the clinical outcome of EAE, mice were treated daily from day 1 post-immunization with 50mg/kg Omomyc via intraperitoneal administration. Mice were monitored daily for clinical signs and weight loss and the clinical evolution was compared to a group of mice that were administered with vehicle (20 mM Na Acetate, pH 5.5, 200 mM sorbitol, 0.02% w/v Tween® 20).

Omomyc treatment was able to significantly ameliorate the clinical course of the disease with animals displaying milder clinical signs (Figure 3). In order to test an administration route compatible with those used in humans, mice were administered intravenously with 75mg/kg of Omomyc thrice a week starting at day 1 post-immunization. The dose of Omomyc was increased to counteract the decreased frequency of administration. Mice were monitored daily for clinical signs and weight loss and the clinical evolution was compared to a group of mice that were administered with vehicle.

The beneficial effect of Omomyc is long lasting (Figure 4). Mice were administered intraperitoneally with 75mg/kg of Omomyc every other day starting at day 1 post-immunization, and then treatment was discontinued after the 4th dose. Mice were followed for 28 days and were monitored daily for clinical signs and weight loss and the clinical evolution was compared to a group of mice that were administered with vehicle.

## Results

All the data has been obtained using the experimental model of multiple sclerosis, experimental autoimmune encephalomyelitis (EAE).

In a first set of experiments, the inventors tested the capacity of Omomyc to modulate the proliferative capacity of splenocytes from EAE-diseased mice. They observed that increasing concentrations of Omomyc abrogated the capacity of proliferation of splenocytes from EAE-diseased mice challenged with a self-antigen (35-55 myelin oligodendrocyte glycoprotein or MOG peptide) or with a polyclonal stimulus (phytohaematoagglutinin or PHA). In addition, the mortality of MOG- and PHA-stimulated EAE splenocytes cultured in the presence of increasing concentrations of Omomyc increased, and both effects were dose-dependent (Figure 1).

The inventors then aimed to test the effect of Omomyc in an *in vivo* approach. Omomyc was administered daily via intraperitoneal injection at a dose of 50mg/kg to EAE mice. Daily treatment with Omomyc was able to delay the onset of the disease and ameliorate the clinical outcome of the disease, since mice developed milder neurological disability, reaching its maximum therapeutic benefit 17 days after the first administration. Loss of weight is also a characteristic feature of EAE-diseased mice that aggravates according to disease severity and is very significant when mice begin to develop neurological disability and reach the peak of the disease. In this regard they also observed that Omomyc-treated animals did not show weight loss compared to their vehicle-treated counterparts, which corroborates that the disease was less severe in Omomyc-treated mice (Figure 2).

The inventors next sought to use a human-approved administration route. To this end, the inventors administered Omomyc intravenously to EAE mice. Since the EAE model is highly sensitive to stress, the frequency of administration was reduced to thrice a week to avoid stress due to daily manipulation of mice. To overcome the decrease in the frequency of Omomyc administration, they used a 75mg/kg dose per administration. Treatment with Omomyc resulted in a significant milder EAE outcome, with a less severe clinical course (AUC of score) and lower neurological disability at the peak of the disease (maximum disability score reached). Indeed, Omomyc-treated animals showed only a mild weight loss with rapid recovery compared to their vehicle-treated counterparts, which displayed a significant and continuous weight loss after the onset of neurological disability (Figure 3).

The inventors also aimed to demonstrate whether the beneficial effect of Omomyc is long lasting. For this purpose, they administered four doses of Omomyc intraperitoneally before EAE onset but after disease induction. They observed a clinical benefit when only four administrations of Omomyc were given to EAE mice. Omomyc-treated mice presented a delay onset of the disease and a milder clinical course (represented as a reduced AUC of daily score). The day of maximum clinical benefit was 14 days after initiation of treatment and seven days after the last dose, meaning that the effect of Omomyc is perdurable. Moreover, mice treated with Omomyc scarcely lost weight compared to the vehicle-treated group of mice (Figure 4).

## Claims

1. A compound selected from the group consisting of:

   (a) a polypeptide comprising the sequence SEQ ID NO: 1 or a functionally equivalent variant thereof,
   (b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,
   (c) a polynucleotide encoding the polypeptide of a) or the conjugate of b),
   (d) a vector comprising the polynucleotide according to c), and
   (e) a cell capable of secreting into the medium the polypeptide according to a) or the conjugate according to b)

   for use in the prevention and/or treatment of an autoimmune disease in a subject.

2. The compound for use according to claim 1, wherein the functionally equivalent variant of SEQ ID NO: 1 is selected from the group consisting of SEQ ID NO: 4, SEQ ID NO: 5, SEQ ID NO; 6, SEQ ID NO: 7, SEQ ID NO: 8, SEQ ID NO: 9, and SEQ ID NO: 10.

3. The compound for use according to any one of claims 1 or 2, wherein the chemical moiety that facilitates the cellular uptake of the polypeptide or the functionally equivalent variant thereof is a cell-penetrating peptide sequence and wherein said cell penetrating peptide sequence and said polypeptide or functionally equivalent variant thereof form a fusion protein.

4. The compound for use according to claim 3, wherein the cell-penetrating peptide sequence is selected from the group consisting of SEQ ID NO: 15, SEQ ID NO: 16, SEQ ID NO: 20, SEQ ID NO: 21, SEQ ID NO: 22, SEQ ID NO: 37 and SEQ ID NO: 38.

5. The compound for use according to any one of claims 1 to 4, wherein the subject is a human.

6. The compound for use according to any one of claims 1 to 5, wherein the compound is a polypeptide comprising the sequence SEQ ID NO: 1.

7. The compound for use according to any one of claims 1 to 6, wherein the autoimmune disease is selected from the group consisting of: celiac disease, diabetes mellitus (type I), Latent Autoimmune Diabetes in Adults (LADA), Graves' disease, inflammatory bowel disease, multiple sclerosis, alopecia areata, Addison's disease, pernicious anemia, psoriasis, rheumatoid arthritis, and systemic lupus erythematosus.

8. The compound for use according to claim 7, wherein the autoimmune disease is multiple sclerosis.

9. The compound for use according to any one of claims 1 to 6, wherein the autoimmune disease is a central nervous system (CNS) autoimmune demyelinating disease, preferably selected from the group consisting of multiple sclerosis (MS), clinically isolated syndrome (CIS), tumefactive (tumor-like) MS, Marburg's acute MS, Balós's concentric sclerosis, acute disseminated encephalomyelitis (ADEM), acute hemorrhagic leukoencephalitis, Schilder's disease, solitary sclerosis, transverse myelitis, Susac's syndrome, leukoaraiosis, myalgic encephalomyelitis, Guillain-Barré syndrome, progressive inflammatory neurophathy, leukodystrophy, including adrenoleukodystrophy, adrenomyeloneuropathy, post-vaccinal encephalitis (PVE), post-infectious encephalomyelitis (PIE), neuromyelitis optica (NMO), optic neuritis and myelin oligodendrocyte glycoprotein (MOG)-associated disease (MOGAD).

10. The compound for use according to any one of claims 1 to 9, wherein the compound is administered intradermally, subcutaneously, intravenously, intraperitoneally, intramuscularly, intranasally, orally or topically, preferably is administered intravenously or intraperitoneally.

11. The compound for use according to any one of claims 1 to 10, wherein the compound is administered in combination with at least another therapeutic agent useful in the treatment of an autoimmune disease, wherein said at least another therapeutic agent is administered simultaneously, sequentially or separately, preferably is selected from the group consisting of corticosteroids, ofatumumab, fingolimod, siponimod, ozanimod, ponesimod, laquinimod, teriflunomide, fumaric acid esters (dimethyl fumarate, diroximel fumarate, monomethyl fumarate), cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig, copaxone, tocilizumab and atacicept..

12. A combination comprising:

   (i) a compound selected from the group consisting of:

   (a) a polypeptide comprising the sequence SEQ ID NO: 1 or a functionally equivalent variant thereof,
   (b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof,
   (c) a polynucleotide encoding the polypeptide of a) or the conjugate of b),
   (d) a vector comprising the polynucleotide according to c), and
   (e) a cell capable of secreting into the medium the polypeptide according to a) or the conjugate according to b); and

   (ii) an agent useful in the treatment of an autoimmune disease selected from the group consisting of copaxone, teriflunomide, fumarate, natalizumab, fingolimod, ocrelizumab, ponesimod, siponimod, ozanimod, and tocilizumab.

13. A pharmaceutical composition comprising a pharmaceutically effective amount of a combination according to claim 12 and a pharmaceutically acceptable excipient.

14. A combination according to claim 12 or a pharmaceutical composition according to claim 13 for use in medicine.

15. A combination according to claim 12 or a pharmaceutical composition according to claim 13 for use in the prevention and/or treatment of an autoimmune disease.

Figure 1

Figure 2

Figure 3

## Clinical score

## Day of disease onset

## Day of best response

## Lose of weight

Figure 4

Europäisches
Patentamt

European
Patent Office

Office européen
des brevets

# EUROPEAN SEARCH REPORT

**Application Number**

EP 24 38 2257

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/087221 A1 (UNIV CALIFORNIA [US]) 28 April 2022 (2022-04-28) * Abstract; paragraphs [0005], [0058], [0070]; claims * | 1-15 | INV. A61K38/16 A61K45/06 A61P25/28 A61P37/06 |
| X | M. R. MCKEOWN ET AL: "Therapeutic Strategies to Inhibit MYC", COLD SPRING HARBOR PERSPECTIVES IN MEDICINE, vol. 4, no. 10, 1 October 2014 (2014-10-01), pages a014266-a014266, XP055695680, DOI: 10.1101/cshperspect.a014266 | 1-7, 10-15 | |
| Y | * Abstract; page 2, column 2 * | 1-15 | |
| Y | US 2016/235807 A1 (SYNERGY PHARMACEUTICALS INC [US]) 18 August 2016 (2016-08-18) * paragraph [0139]; claims * | 1-15 | |
| Y | US 2020/291100 A1 (SINCLAIR DAVID A [US] ET AL) 17 September 2020 (2020-09-17) * paragraphs [0009], [0010], [0122] * | 1-15 | TECHNICAL FIELDS SEARCHED (IPC) A61K A61P |
| X | WO 2020/187998 A1 (FUNDACIO PRIVADA INST DINVESTIGACIO ONCOLÒGICA DE VALL HEBRON [ES] ET) 24 September 2020 (2020-09-24) | 12-14 | |
| Y | * page 33, lines 22-28; claims * | 1-15 | |
| Y | WO 2023/076424 A2 (ZENG WEI PING [US]) 4 May 2023 (2023-05-04) * paragraphs [0002], [0003], [0145], [0146]; figure 7a * | 1-15 | |

The present search report has been drawn up for all claims

| Place of search | Date of completion of the search | Examiner |
|---|---|---|
| Munich | 2 August 2024 | Langer, Astrid |

EPO FORM 1503 03.82 (P04C01)

## ANNEX TO THE EUROPEAN SEARCH REPORT
## ON EUROPEAN PATENT APPLICATION NO.

EP 24 38 2257

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2024

| Patent document cited in search report | Publication date | Patent family member(s) | Publication date |
|---|---|---|---|
| WO 2022087221 A1 | 28-04-2022 | AU 2021365153 A1 | 25-05-2023 |
| | | CA 3195795 A1 | 28-04-2022 |
| | | EP 4232061 A1 | 30-08-2023 |
| | | JP 2023549476 A | 27-11-2023 |
| | | KR 20230092938 A | 26-06-2023 |
| | | US 2023382953 A1 | 30-11-2023 |
| | | WO 2022087221 A1 | 28-04-2022 |
| US 2016235807 A1 | 18-08-2016 | AU 2014331812 A1 | 28-04-2016 |
| | | AU 2019202706 A1 | 16-05-2019 |
| | | CA 2926685 A1 | 16-04-2015 |
| | | EP 3065757 A2 | 14-09-2016 |
| | | JP 2016534043 A | 04-11-2016 |
| | | JP 2020015746 A | 30-01-2020 |
| | | US 2016235807 A1 | 18-08-2016 |
| | | US 2020113968 A1 | 16-04-2020 |
| | | WO 2015054500 A2 | 16-04-2015 |
| US 2020291100 A1 | 17-09-2020 | AU 2013329312 A1 | 28-05-2015 |
| | | AU 2018271373 A1 | 20-12-2018 |
| | | US 2015266946 A1 | 24-09-2015 |
| | | US 2018118819 A1 | 03-05-2018 |
| | | US 2020291100 A1 | 17-09-2020 |
| | | WO 2014059029 A1 | 17-04-2014 |
| WO 2020187998 A1 | 24-09-2020 | AU 2020242284 A1 | 16-09-2021 |
| | | BR 112021018506 A2 | 30-11-2021 |
| | | CA 3133155 A1 | 24-09-2020 |
| | | CN 113795264 A | 14-12-2021 |
| | | EA 202192555 A1 | 25-11-2021 |
| | | EP 3941503 A1 | 26-01-2022 |
| | | IL 286473 A | 01-12-2021 |
| | | JP 2022528020 A | 07-06-2022 |
| | | KR 20220012839 A | 04-02-2022 |
| | | SG 11202109066R A | 29-09-2021 |
| | | TW 202102542 A | 16-01-2021 |
| | | US 2022152179 A1 | 19-05-2022 |
| | | WO 2020187998 A1 | 24-09-2020 |
| | | ZA 202107947 B | 25-10-2023 |
| WO 2023076424 A2 | 04-05-2023 | CN 118175996 A | 11-06-2024 |
| | | US 2023135127 A1 | 04-05-2023 |
| | | WO 2023076424 A2 | 04-05-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019018898 A
- WO 2006135436 A
- US 6395713 B
- WO 9402595 A
- US 20010007666
- US 2003077829
- WO 0347518 A, Wang
- WO 0346185 A
- US 6447796 B
- US 2002130430
- WO 0053722 A
- US 20030077829
- EP 52322 A
- EP 36676 A
- EP 88046 A
- EP 143949 A

**Non-patent literature cited in the description**

- **MARTIN R et al.** Immunological aspects of demyelinating diseases. *Ann Rev Immunol.*, 1992, vol. 10, 153-187
- **GREEN ; WUTS**. Protecting Groups in Organic Synthesis. John Wiley and Sons, 1991
- **DANG ; LEE**. *Mol.Cell. Biol*, 1988, vol. 8, 4048-4054
- **NAIR, S. K ; BURLEY, S. K.** *Cell*, 2003, vol. 112, 193-205
- **ALTSCHUL, S et al.** *J. Mol. Biol.*, 1990, vol. 215, 403-410
- **DORDO et al.** *J. Mol. Biol*, 1999, vol. 217, 721-739
- **TAYLOR et al.** *J. Theor. Biol.*, 1986, vol. 119, 205-218
- **THOMPSON et al.** *Nucleic Acids Res*, 1994, vol. 22, 4673-4680
- **SOUCEK et al.** *Oncogene*, 1998, vol. 17, 2463-2472
- **GORLICH D.** *EMBO*, 1998, vol. 5 (17), 2721-7
- **TERPE K.** *Appl. Microbiol. Biotechnol*, 2003, vol. 60, 523-525
- **BAXTER AG**. The origin and application of experimental autoimmune encephalomyelitis. *Nat Rev Immunol*, 2007, vol. 7, 904-912
- **STORCH MK et al.** Autoimmunity to myelin oligodendrocyte glycoprotein in rats mimics the spectrum of multiple sclerosis pathology. *Brain Pathol.*, 1998, vol. 8 (4), 681-94
- **HIRANO Y**. Acute disseminated encephalomyelitis. *Nippon Rinsho.*, 1997, vol. 55 (4), 934-9
- **LUBLIN FD et al.** Defining the clinical course of multiple sclerosis: the 2013 revisions. *Neurology*, 15 July 2014, vol. 83 (3), 278-86
- Remington's Pharmaceutical Sciences. Mack Publishing Company, 1995
- **FAULI ; TRILLO C**. Tratado de Farmacia Galénica. Luzán 5, 1993
- Remington: The Science and Practice of Pharmacy. Lippincott Williams & Wilkins, 2003
- **ULMER et al.** *Science*, 1993, vol. 259, 1745-49
- **COHEN**. *Science*, 1993, vol. 259, 1691-1692
- **AKHTAR et al.** *Trends Cell Bio*, 1992, vol. 2, 139
- Delivery Strategies for Antisense Oligonucleotide Therapeutics. 1995
- **MAURER et al.** *Mol. Membr. Biol*, 1999, vol. 16, 129-40
- **HOFLAND ; HUANG**. *Handb. Exp. Pharmacol*, 1999, vol. 137, 165-92
- **LEE et al.** *ACS Symp. Ser*, 2000, vol. 752, 184-92
- **SELBO et al.** *Int. J. Cancer*, 2000, vol. 87, 853-59
- **SELBO et al.** *Tumour Biol.*, 2002, vol. 23, 103-12
- **GONZALEZ et al.** *Bioconjug. Chem.*, 1999, vol. 10, 1068-74
- Nonviral Vectors for Gene Therapy. Academic Press, 1999
- **GOODMAN**. Goldman's The Pharmacological Basis of Therapeutics. 1996, 1707-1711
- **GOODMAN**. Goldman's The Pharmacological Basis of Therapeutics. 2001, 475-493
- **REAGAN-SHAW S. et al.** Dose translation from animal to human studies revisited''. *FASEB J*, 2008, vol. 22 (3), 659-661
- **REAGAN-SHAW S. et al.** Dose translation from animal to human studies revisited. *FASEB J*, 2008, vol. 22 (3), 659-661
- **EPSTEIN et al.** *Proc. Natl. Acad. Sci. USA*, 1985, vol. 82, 3688-3692

- **HWANG et al.** *Proc. Natl. Acad. Sci. USA*, 1980, vol. 77, 4030-4034
- **GUTIERREZ-FRANCO A et al.** Semaphorin 7A as a Potential Therapeutic Target for Multiple Sclerosis. *Mol Neurobiol*, 2017, vol. 54 (6), 4820-4831
- **BAKER D.** ; **AMOR S.** Publication guidelines for refereeing and reporting on animal use in experimental autoimmune encephalomyelitis. *J Neuroimmunol*, 2012, vol. 242 (1-2), 78-83